(19) Europäisches Patentamt
European Patent Office
Office européen des brevets

(11) Veröffentlichungsnummer: **0 071 935**
**B1**

(12) # EUROPÄISCHE PATENTSCHRIFT

(45) Veröffentlichungstag der Patentschrift: **13.11.85**

(21) Anmeldenummer: **82106962.2**

(22) Anmeldetag: **02.08.82**

(51) Int. Cl.⁴: **C 07 D 209/42**, C 07 D 401/12, C 07 D 413/02, C 07 D 417/12, C 07 D 403/12, A 61 K 31/40

(54) **1-Phenyl-2-aminocarbonylindol-Verbindungen sowie Verfahren und Zwischenprodukte zu ihrer Herstellung und diese Verbindungen enthaltende Arzneimittel.**

(30) Priorität: **08.08.81 DE 3131527**

(43) Veröffentlichungstag der Anmeldung: **16.02.83 Patentblatt 83/7**

(45) Bekanntmachung des Hinweises auf die Patenterteilung: **13.11.85 Patentblatt 85/46**

(84) Benannte Vertragsstaaten: **AT BE CH DE FR GB IT LI LU NL SE**

(56) Entgegenhaltungen:
**EP - A - 0 025 501**
**DE - A - 105 495**
**DE - A - 158 089**
**DE - A - 1 720 033**
**FR - A - 1 503 908**
**FR - A - 2 260 332**
**FR - M - 1 527**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH, Hans-Böckler-Allee 20 Postfach 220, D-3000 Hannover 1 (DE)**

(72) Erfinder: **Ohlendorf, Heinrich-W. Dipl.-Chem. Dr. rer. nat., Schumachersweg 18, D-3008 Garbsen 2 (DE)**
Erfinder: **Kaupmann, Wilhelm Dipl.-Chem. Dr. Ing., St. Ingbert-Weg 9, D-3000 Hannover-Kirchrode (DE)**
Erfinder: **Kühl, Ulrich, Dr., Franz-Burger-Strasse 10, D-3007 Gehrden (DE)**
Erfinder: **Buschmann, Gerd, Dr., Matthäikirchstrasse 27, D-3000 Hannover (DE)**
Erfinder: **Magda, Stephen John, Dipl.-Biologe, Dr., Gellertstrasse 24, D-3000 Hannover 1 (DE)**

(74) Vertreter: **Lauer, Dieter, Dr., c/o Kali-Chemie AG Postfach 220 Hans-Böckler-Allee 20, D-3000 Hannover 1 (DE)**

**EP 0 071 935 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1-Phenyl-2-aminoalkylaminocarbonylindol-Verbindungen und deren Salze sowie diese Verbindungen enthaltende pharmazeutische Zubereitungen und Verfahren und Zwischenprodukte zur Herstellung dieser Verbindungen.

Das belgische Patent Nr. 701 023 und das französische Patent Nr. 1 503 908 beschreiben 2-Carboxamidoindol-Verbindungen, welche antiemetische und zentraldämpfende Wirkungen zeigen, und die französischen Patente Nr. 1 527 M und 1 313 759 beschreiben 2-Carboxamidoindol-Verbindungen, welche ebenfalls antiemetische und daneben lokalanästhesierende und antifibrillatorische Eigenschaften besitzen.

Der Erfindung liegt die Aufgabe zugrunde, neue 1-Phenyl-2-aminocarbonylindol-Verbindungen mit wertvollen pharmakologischen Eigenschaften zu entwickeln.

Es wurde nun gefunden, daß die neuen 1-Phenyl-2-aminocarbonylindol-Verbindungen wertvolle pharmakologische Wirkungen, insbesondere antiarrhythmische Wirkungen, besitzen und ein vorteilhaftes Wirkungsprofil mit guter therapeutischer Breite und geringer Toxizität aufweisen. Aufgrund dieser Wirkungen sind die neuen Verbindungen als Arzneimittel, insbesondere zur Behandlung von Herz-Rhythmusstörungen, geeignet.

Die vorliegende Erfindung betrifft daher neue 1-Phenyl-2-aminocarbonylindol-Verbindungen der allgemeinen Formel I

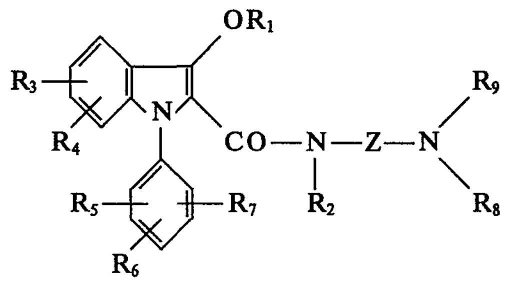

(I)

worin

$R_1$ Wasserstoff, eine Alkyl-, Alkenyl-, Cycloalkyl- oder Cycloalkylalkylgruppe mit bis zu 7 Kohlenstoffatomen bedeutet,
$R_2$ Wasserstoff oder $C_1$—$C_4$-Alkyl bedeutet,
$R_3$ Wasserstoff, Halogen, $C_1$—$C_4$-Alkyl, Hydroxy oder $C_1$—$C_4$-Alkoxy bedeutet,
$R_4$ Wasserstoff, Halogen, $C_1$—$C_4$-Alkyl, $C_1$—$C_4$-Alkoxy, Hydroxy oder, falls $R_3$ Wasserstoff ist, auch Nitro oder Trifluormethyl bedeutet, oder
$R_3$ und $R_4$ an benachbarte Kohlenstoffatome gebunden sind und zusammen Methylendioxy oder Äthylendioxy bedeuten,
$R_5$ Wasserstoff, $C_1$—$C_4$-Alkyl, Halogen, Hydroxy oder $C_1$—$C_4$-Alkoxy bedeutet,
$R_6$ Wasserstoff, $C_1$—$C_4$-Alkyl, $C_1$—$C_4$-Alkoxy, Halogen, Hydroxy oder, falls $R_5$ Wasserstoff ist, auch Nitro oder Trifluormethyl bedeutet, oder
$R_5$ und $R_6$ an benachbarte Kohlenstoffatome gebunden sind und zusammen Methylendioxy oder Äthylendioxy bedeuten,
$R_7$ Wasserstoff, oder, falls $R_5$ und $R_6$ $C_1$—$C_4$-Alkoxy sind, auch $C_1$—$C_4$-Alkoxy bedeutet,
$R_8$ Wasserstoff oder $C_1$—$C_4$-Alkyl bedeutet,
$R_9$ Wasserstoff oder $C_1$—$C_4$-Alkyl bedeutet, oder
$R_8$ und $R_9$ zusammen mit dem Stickstoffatom, an das sie gebunden sind, eine heterocyclische Gruppe a bilden

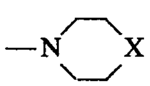

(a)

worin X für eine Bindung, $-CH_2-$, $-C_2H_4-$, O oder S steht,
Z eine Alkylenkette mit 2—5 Kohlenstoffatomen bedeutet, welche gegebenenfalls an einem nicht an Stickstoff gebundenen Kohlenstoff durch Hydroxy substituiert ist,

sowie deren Säureadditionssalze.

Sofern in den Verbindungen der Formel I die Substituenten $R_3$ bis $R_7$ der Phenylringe eine niedere Alkylgruppe enthalten, kann diese gerade oder verzweigt sein und 1—4 Kohlenstoffatome enthalten. So kommen insbesondere Methyl, Äthyl, n-Propyl, Isopropyl, n-Butyl oder tert.-Butyl, bevorzugt Methyl oder Äthyl in Frage. Insbesondere bei Di- und Trisubstitutionen an den Phenylringen stellen alkylhaltige Substituenten vorzugsweise Methyl oder Methoxy dar.

Als Halogensubstituenten in den Phenylringen kommen insbesondere Fluor, Chlor oder Brom, bevorzugt Chlor oder Brom, in Frage.

Die Substituenten $R_3$ und $R_4$ stellen vorzugsweise Wasserstoff oder Halogen dar. Die Substituenten $R_5$—$R_7$ stellen vorzugsweise Wasserstoff, Halogen oder niederes Alkyl dar.

Der Substituent $R_1$ steht für Wasserstoff oder gerades, verzweigtes oder cyclisches Alkyl oder Alkenyl mit bis zu 7 Kohlenstoffatomen. Insbesondere stellt er Wasserstoff, niederes Alkyl, vorzugsweise Methyl, oder Cycloalkylalkyl dar. Als niedere Alkylgruppen kommen gerade oder verzweigte Alkylgruppen, welche 1—4 Kohlenstoffatome enthalten können, in Frage. Als Beispiele für niedere Alkyl- und Cycloalkylalkylgruppen seien genannt Methyl, Äthyl, n-Propyl, Isopropyl, tert.-Butyl oder Cyclopropylmethyl.

Der Substituent $R_2$ steht insbesondere für Wasserstoff. Sofern $R_2$ niederes Alkyl bedeutet enthält dieses 1—4 Kohlenstoffatome. Es kommen die für $R_1$ genannten Gruppen, insbesondere Methyl oder Äthyl, in Frage.

Falls $R_8$ und/oder $R_9$ eine niedere Alkylgruppe bedeuten, kann diese gerade oder verzweigt sein und 1—4 Kohlenstoffatome enthalten. Als Alkylgruppen kommen insbesondere Methyl-, Äthyl-, Propyl- und Butyl-Gruppen in Frage. Insbesondere steht die $NR_8R_8$-Gruppe für eine vorzugsweise unverzweigte Dialkylaminogruppe, beispielsweise die Diäthylamino-Gruppe.

Z stellt eine Alkylenkette mit 2—5 Kohlenstoffatomen dar, vorzugsweise eine gerade Alkylenkette mit 2—4 Kohlenstoffatomen. Falls Z eine durch Hydroxy substituierte Alkylenkette bedeutet, stellt diese vorzugsweise die 2-Hydroxypropylen-Kette dar.

Erfindungsgemäß werden die neuen 1-Phenyl-2-aminocarbonylindol-Verbindungen der Formel I und deren Säureadditionssalze erhalten, indem man in an sich bekannter Weise

a) Verbindungen der Formel II

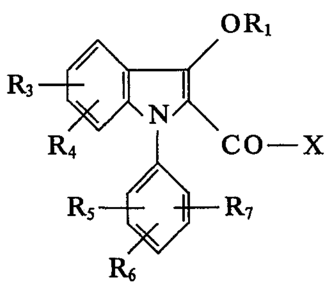

(II)

worin $R_1$, $R_3$, $R_4$, $R_5$, $R_6$ und $R_7$ obige Bedeutung besitzen und X Hydroxy oder eine reaktive Gruppe bedeutet,

mit einer Verbindung der Formel VI

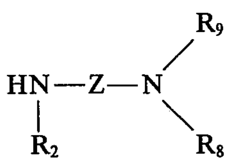

(VI)

worin $R_2$, $R_8$, $R_9$ und Z obige Bedeutung besitzen, umsetzt, oder

b) Verbindungen der Formel III

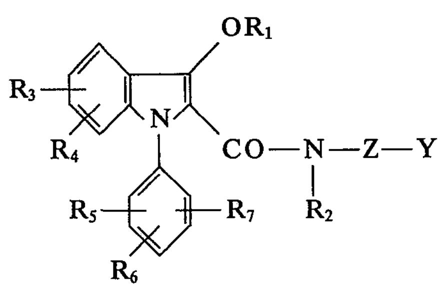

(III)

worin $R_1$, $R_2$, $R_3$, $R_4$, $R_5$, $R_6$, $R_7$ und Z obige Bedeutung besitzen, und Y eine aminolytisch abspaltbare Gruppe bedeutet,

mit einer Verbindung der Formel VII

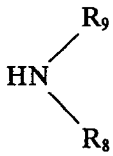

(VII)

worin $R_8$ und $R_9$ obige Bedeutung besitzen, umsetzt, oder

c) Verbindungen der Formel IVa

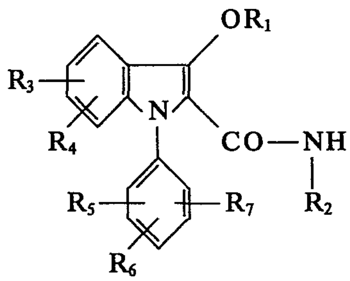

(IVa)

worin $R_1$, $R_2$, $R_3$, $R_4$, $R_5$, $R_6$ und $R_7$ obige Bedeutung besitzen,

mit Verbindungen der Formel VIII

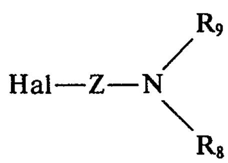

(VIII)

worin Z, $R_8$ und $R_9$ obige Bedeutung besitzen, und Hal Halogen bedeutet,

umsetzt, oder

d) zur Herstellung von Verbindungen der Formel Ia

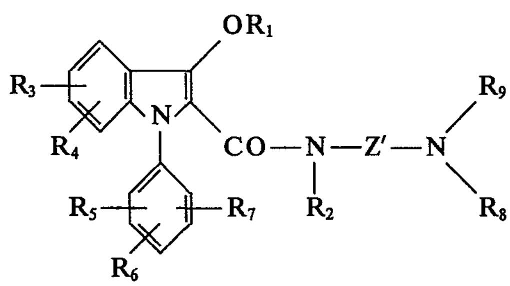

(Ia)

worin $R_1$, $R_2$, $R_3$, $R_4$, $R_5$, $R_6$, $R_7$, $R_8$ und $R_9$ obige Bedeutung besitzen, und Z' eine Alkylengruppe mit 2–5 Kohlenstoffatomen bedeutet, welche an einem nicht an Stickstoff gebundenen Kohlenstoffatom durch Hydroxy substituiert ist,

Verbindungen der Formel IV

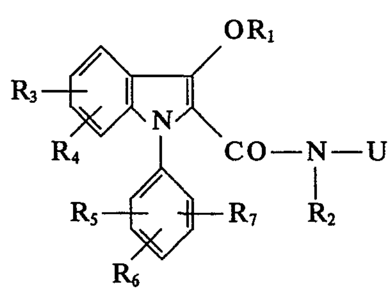

(IV)

worin $R_1$, $R_2$, $R_3$, $R_4$, $R_5$, $R_6$ und $R_7$ obige Bedeutung besitzen und U für Wasserstoff oder eine Gruppe der Formel V

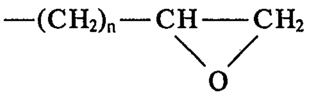

(V)

steht, worin n 1—3 bedeutet,

umsetzt mit einer Verbindung der Formel IX

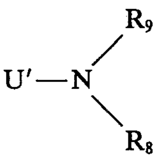

(IX)

worin $R_8$ und $R_9$ obige Bedeutung besitzen, und U', falls U Wasserstoff darstellt, für eine Gruppe der Formel V, oder, falls U eine Gruppe der Formel V darstellt, für Wasserstoff steht,

und gegebenenfalls freie Verbindungen der Formel I in ihre Säureadditionssalze überführt oder die Säureadditionssalze in die freien Verbindungen der Formel I überführt.

Die Umsetzung der Säuren oder Säurederivate der Formel II mit Aminen der Formel VI kann nach an sich zur Bildung von Amid-Gruppierungen durch Aminoacylierung üblichen Methoden ausgeführt werden. Es können die Säuren der Formel II (X = OH) oder deren reaktionsfähigen Derivate, worin X eine reaktionsfähige Gruppe bedeutet, eingesetzt werden. Als reaktionsfähige Derivate kommen insbesondere Säurehalogenide, vorzugsweise -chloride, Ester und gemischte Anhydride der Säuren der Formel II in Frage, z. B. Verbindungen der Formel II, worin die reaktive Gruppe X Halogen, insbesondere Chlor oder Brom, niederes Alkoxy, insbesondere Alkoxy mit 1—4 Kohlenstoffatomen, oder eine Gruppe O— CO—W bedeutet, worin W für niederes Alkyl oder niederes Alkoxy steht. Die Acylierung kann in einem unter den Reaktionsbedingungen inerten Lösungsmittel bei Temperaturen zwischen Raumtemperatur und dem Siedepunkt des Lösungsmittels erfolgen. Als Lösungsmittel eignen sich halogenierte Kohlenwasserstoffe wie Methylenchlorid oder Chloroform, aromatische Kohlenwasserstoffe wie Benzol, Toluol, Xylol oder Chlorbenzol, cyclische Äther wie Tetrahydrofuran oder Dioxan, Dimethylformamid oder Gemische dieser Lösungsmittel.

Die Acylierung kann gegebenenfalls, insbesondere wenn ein Säurehalogenid oder -anhydrid der Formel II verwendet wird, in Gegenwart eines säurebindenden Reagenzes durchgeführt werden. Als säurebindende Mittel eignen sich anorganische Basen, insbesondere Alkalimetallcarbonate und -hydroxyde wie zum Beispiel Natrium- oder Kaliumcarbonat oder Kaliumhydroxyd oder organische Basen, insbesondere tertiäre Niederalkylamine und Pyridine wie z. B. Triäthylamin, Tripropylamin, Tributylamin, Pyridin, 4-Dimethylaminopyridin oder 4-Pyrrolidinpyridin. Statt einer Fremdbase kann auch ein Überschuß des Amins der Formel VI verwendet werden. Im Überschuß eingesetzte organische Basen können gleichzeitig auch als Lösungsmittel dienen.

Falls eine Säure der Formel II selbst oder auch ein Ester davon eingesetzt wird, so wird die Umsetzung der Säure oder auch des Esters mit dem Amin der Formel VI zweckmäßigerweise in Gegenwart eines aus der Peptidchemie als zur Amidbildung geeignet bekannten Kopplungsreagenzes durchgeführt. Als Beispiele von Kopplungsreagenzien, welche die Amidbildung der freien Säuren dadurch fördern, daß sie mit der Säure in situ unter Bildung eines reaktionsfähigen Säurederivates reagieren, seien insbesondere genannt Alkyl-, vorzugsweise Cycloalkylcarbodiimide, vorzugsweise Dicyclohexylcarbodiimid, Carbonyldiimidazol und N-Niederalkyl-2-halogenpyridiniumsalze, insbesondere Halogenide oder Tosylate, vorzugsweise N-Methyl-2-chlorpyridiniumjodid (siehe z. B. Mukayama in Angew. Chemie 91 789—812). Die Umsetzung in Gegenwart eines Kopplungsreagenzes kann zweckmäßig bei Temperaturen von −30 bis +30°C unter Benutzung von Lösungsmitteln wie halogenierten Kohlenwasserstoffen und/oder aromatischen Lösungsmitteln gegebenenfalls in Gegenwart eines säurebindenden Amins durchgeführt werden. Als Beispiele von Kopplungsreagenzien, welche die Amidbildung der Ester durch Bildung eines reaktionsfähigen Derivates der Aminoverbindung fördern, seien genannt Tri-Niederalkylaluminium-Verbindungen, insbesondere Trimethylaluminium, welches zur Aktivierung der Umsetzung der Aminoverbindungen mit Estern geeignet ist, oder $PCl_3$. Als inerte Lösungsmittel für die Umsetzung in Gegenwart von Trialkylaluminium eignen sich insbesondere aromatische Kohlenwasserstoffe und/oder halogenierte Kohlenwasserstoffe. Die Umsetzung der Aminoverbindung mit dem Trialkylaluminium wird vorzugsweise bei Temperaturen von −20°C bis Raumtemperatur durchgeführt. Die anschließende Reaktion der intermediär gebildeten Monoalkylaluminiumazo-Verbindungen mit dem Ester kann insbesondere bei Temperaturen zwischen Raumtemperatur und Siedepunkt des Lösungsmittels durchgeführt werden. Weitere zur erfindungsgemäßen Amidbildung geeignete Kopplungsreagenzien,

welche auch in Peptidsynthesen Anwendung finden, sind z. B. bekannt aus Advanced Organic Chemistry by Jerry March, McGraw-Hill Ltd., 2. Ausgabe, Seite 382–388 und The Chemistry of Amides, by Jacob Zabicky 1970, Interscience Publishers, John Wiley & Sons, London, Kapitel 2: Synthesis of Amides.

Falls die Ausgangsverbindungen freie Hydroxy-Gruppe enthalten, können diese gewünschtenfalls vor der Umsetzung in an sich bekannter Weise mit einer Schutzgruppe versehen werden, welche anschließend leicht wieder abspaltbar ist. Geeignete nach der Reaktion leicht wieder abspaltbare Schutzgruppen sind z. B. bekannt aus E. McOmie »Protective Groups in Organic Chemistry« Plenum Press 1971. Beispielsweise eignen sich zum Schutz einer Hydroxylgruppe Ester, z. B. Acetate, und leicht spaltbare Äther, insbesondere Tetrahydropyranyläther.

Die Umsetzung von Verbindungen der Formel III mit Aminen der Formel VII kann nach an sich zur Aminoalkylierung üblichen Methoden ausgeführt werden. Zweckmäßigerweise wird die Umsetzung bei erhöhter Temperatur, beispielsweise bei Temperaturen zwischen 50 und 150°C, unter basischen Bedingungen durchgeführt. Als aminolytisch abspaltbare Reste in Verbindungen der Formel III kommen insbesondere Halogene wie Chlor, Brom, Jod oder auch organische Sulfonsäurereste in Frage, insbesondere Reste von Niederalkansulfonsäuren wie z. B. Methansulfonsäure oder Äthansulfonsäure oder von aromatischen Sulfonsäuren, insbesondere Benzolsulfonsäure oder durch niederes Alkyl substituierte Benzolsulfonsäuren, z. B. Toluolsulfonsäuren, oder durch Halogen substituierte Benzolsulfonsäuren wie z. B. Bromsulfonsäuren. Zweckmäßigerweise wird die Reaktion in einem unter den Reaktionsbedingungen inerten organischen Lösungsmittel durchgeführt. Als Beispiele geeigneter Lösungsmittel seien genannt aromatische Kohlenwasserstoffe wie Benzol, Toluol oder Xylol, cyclische Äther wie Dioxan, Dimethylformamid, 1,3-Dimethyl-2-imidazolidinon, Hexamethylphosphortriamid, Sulfolan, Dimethylsulfoxyd, Tetramethylharnstoff oder niedere Alkylalkohole, beispielsweise Isopentanol. Gewünschtenfalls kann die Umsetzung der Verbindungen der Formel III mit den Aminen der Formel VII jedoch auch ohne Lösungsmittel in der Schmelze stattfinden. Zweckmäßig kann man die Reaktion unter Zusatz einer organischen oder anorganischen Base durchführen. Man kann jedoch auch einen Überschuß der Verbindung der Formel VII verwenden und diesen als interne Base benutzen. Geeignete anorganische Basen sind insbesondere Alkalimetallcarbonate oder -bicarbonate wie Natriumcarbonat, Natriumbicarbonat oder Kaliumcarbonat. Als organische Basen sind tertiäre organische Amine geeignet, insbesondere tertiäre Niederalkylamine wie Triäthylamin, n-Tripropylamin, n-Tributylamin oder 1,4-Dimethylpiperazin.

Die Umsetzung der Amide der Formel IVa mit den Alkylhalogenidverbindungen der Formel VIII kann auf an sich bekannte Weise erfolgen. Zweckmäßigerweise wird die Umsetzung in einem unter den Reaktionsbedingungen inerten Lösungsmittel unter Zusatz einer organischen oder anorganischen Base, beispielsweise einer der vorgehend genannten Basen, bei erhöhter Temperatur, beispielsweise bei Temperaturen zwischen 50 und 120°C, vorzugsweise Siedetemperatur des Lösungsmittels, durchgeführt. Als inerte Lösungsmittel eignen sich beispielsweise cyclische Äther wie Dioxan oder Tetrahydrofuran, Dimethylformamid oder Niederalkylketone wie Aceton.

Die Umsetzung von Verbindungen der Formel IV mit Verbindungen der Formel IX kann auf an sich zur Umsetzung von Epoxiden übliche Weise erfolgen. Zweckmäßigerweise wird die Umsetzung in einem unter den Reaktionsbedingungen inerten Lösungsmittel bei Temperaturen zwischen Raumtemperatur und 100°C durchgeführt. Als Lösungsmittel eignen sich beispielsweise aromatische Kohlenwasserstoffe wie Benzol, Toluol oder Xylol, offene oder cyclische Äther wie z. B. Diäthyläther, Tetrahydrofuran oder Dioxan oder niedere Alkohole wie z. B. Isopropanol.

Falls die Verbindungen der Formel III, IVa oder IV freie Hydroxygruppen enthalten, werden diese zweckmäßigerweise während der vorstehenden Umsetzungen in an sich bekannter Weise mit einer Schutzgruppe versehen.

Verbindungen der Formel I, worin Z eine Hydroxygruppe enthält, werden bei der Synthese in Form ihrer Racemate erhalten. Unter den Schutz dieser Erfindung fallen die racemischen Gemische wie auch die optisch aktiven Formen dieser Verbindungen. Die optisch aktiven Verbindungen können aus den racemischen Gemischen in an sich bekannter Weise durch Umsetzung mit geeigneten optisch aktiven Säuren, wie beispielsweise Weinsäure, O,O'-Dibenzoyl-Weinsäure, Mandelsäure, Di-O-isopropyliden-2-oxo-L-gulonsäure, und anschließende fraktionierte Kristallisation der gewonnenen Salze in ihre optisch aktiven Antipoden aufgetrennt werden.

Die Verbindungen der Formel I können auf an sich bekannte Weise aus dem Reaktionsgemisch isoliert und gereinigt werden. Säureadditionssalze können in üblicher Weise in die freien Basen überführt werden, und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit Chlorwasserstoffsäure, Bromwasserstoffsäure, Schwefelsäure, Phosphorsäure, Methansulfonsäure, Äthanolsulfonsäure, Cyclohexylaminosulfonsäure, Amidosulfonsäure, Essigsäure, Milchsäure, Weinsäure, Phenylessigsäure oder Mandelsäure.

Verbindungen der Formel II stellen wertvolle Zwischenprodukte für die Herstellung von pharmakologisch aktiven Verbindungen, beispielsweise den Verbindungen der Formel I, dar.

Verbindungen der Formel IIa

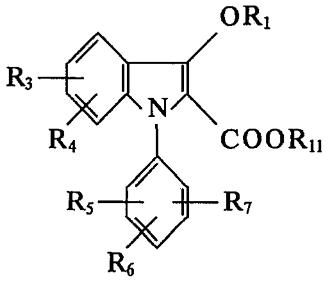

(IIa)

worin $R_1$, $R_3$, $R_4$, $R_5$, $R_6$ und $R_7$ obige Bedeutung besitzen und $R_{11}$ niederes Alkyl bedeutet, können auf an sich bekannte Weise erhalten werden, indem man

a') Verbindungen der Formel X

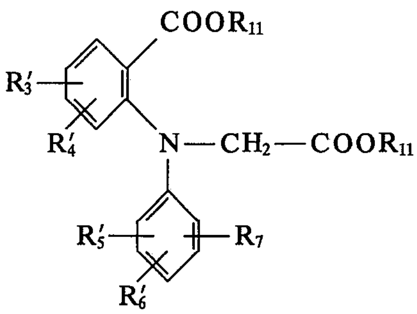

(X)

worin $R_7$ und $R_{11}$ obige Bedeutung besitzen und $R_3'$, $R_4'$, $R_5'$ und $R_6'$ jeweils die für $R_3$, $R_4$, $R_5$ und $R_6$ angegebene Bedeutung besitzen, wobei jedoch eine freie Hydroxygruppe mit einer Schutzgruppe versehen ist, in an sich bekannter Weise cyclisiert zu Verbindungen der Formel IIb

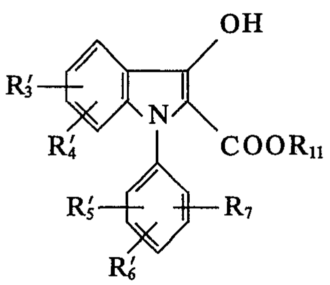

(IIb)

worin $R_3'$, $R_4'$, $R_5'$, $R_6'$, $R_7$ und $R_{11}$ obige Bedeutung besitzen, und diese gewünschtenfalls zu Verbindungen der Formel IIc

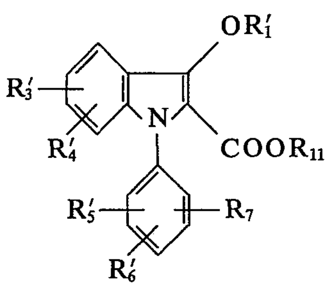

(IIc)

worin $R_3'$, $R_4'$, $R_5'$, $R_6'$, $R_7$ und $R_{11}$ obige Bedeutung besitzen und $R_1'$ die für $R_1$ angegebene Bedeutung mit Ausnahme von Wasserstoff besitzt, veräthert und allfällige Hydroxyschutzgruppen in an sich bekannter Weise wieder abspaltet, oder

b') zur Herstellung von Verbindungen der Formel IIa

Verbindungen der Formel XI

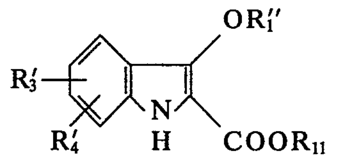

(XI)

worin $R_3'$, $R_4'$ und $R_{11}$ obige Bedeutung besitzen, und $R_1''$ die für $R_1'$ angegebene Bedeutung besitzt oder eine niedere Acylschutzgruppe darstellt,

mit Verbindungen der Formel XII

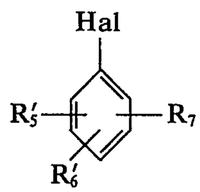

(XII)

worin $R_5'$, $R_6'$, $R_7$ und Hal obige Bedeutung besitzen, umsetzt und eine allfällige Acylschutzgruppe $R_1''$ und/oder allfällige Hydroxyschutzgruppen auf an sich bekannte Weise abspaltet.

Die Verbindungen der Formel IIa können anschließend auf an sich bekannte Weise zu den entsprechenden Säuren hydrolisiert und diese in an sich bekannter Weise in weitere reaktionsfähige Säurederivate überführt werden. Die Hydrolyse der Ester der Formel IIa zu den entsprechenden Säuren kann nach an sich zur Esterhydrolyse üblichen Methoden in alkalischem oder saurem Medium durchgeführt werden, beispielsweise durch Erhitzen der Ester in wäßriger Alkalimetallhydroxyd-Lösung, zweckmäßigerweise in Gegenwart eines mit Wasser mischbaren inerten organischen Lösungsmittels, beispielsweise eines niederen Alkohols. Die Überführung der freien Säuren der Formel II in reaktive Säurederivate erfolgt ebenfalls auf an sich bekannte Weise. So können Säurehalogenide der Formel II zum Beispiel durch Umsetzung der Säuren mit einem anorganischen Säurehalogenid, beispielsweise Phosphortrichlorid, Phosphorpentachlorid, Phosphortribromid oder Thionylchlorid, erhalten werden. Gewünschtenfalls kann die Umsetzung in Gegenwart von Pyridin oder einer anderen tertiären organischen Base durchgeführt werden. Gemischte Säureanhydride können zum Beispiel durch Umsetzung von Alkalimetallsalzen der Säuren der Formel II mit einem entsprechenden organischen Säurechlorid in Gegenwart einer tertiären organischen Base, beispielsweise Pyridin, erhalten werden.

Die Cyclisierung der Verbindungen der Formel X gemäß Verfahrensvariante a') wird zweckmäßigerweise in einem unter den Reaktionsbedingungen inerten Lösungsmittel in Gegenwart einer starken Base bei erhöhter Temperatur, beispielsweise bei Temperaturen zwischen 50 und 150°C, durchgeführt. Als starke Basen eignen sich beispielsweise Alkalimetallhydride wie Natriumhydrid oder niedere Alkalimetallalkoholate wie z. B. Natriummethylat. Als inerte Lösungsmittel eignen sich beispielsweise aromatische Kohlenwasserstoffe wie Toluol oder Xylol, niedere Alkohole, Dimethylformamid oder Mischungen solcher Lösungsmittel. So sind beispielsweise bei Verwendung von Alkalimetallhydriden aromatische Kohlenwasserstoffe oder Dimethylformamid und bei Alkalimetallalkoholaten die entsprechenden Alkohole als Lösungsmittel besonders geeignet. Bei der Umsetzung fällt die Verbindung der Formel IIb in Form ihres Alkalimetallsalzes an und kann bei der Aufarbeitung durch Ansäuern des Reaktionsgemisches in Freiheit gesetzt werden.

Die Verätherung von Verbindungen der Formel IIb zu Verbindungen der Formel IIc kann nach an sich bekannter Methoden erfolgen, beispielsweise in dem man die Verbindung der Formel IIb in Gegenwart einer Base mit einer Verbindung der Formel XIII

$$R_1'—X_1 \qquad \text{(XIII)}$$

worin $R_1'$ obige Bedeutung besitzt und $X_1$ für Halogen, vorzugsweise Chlor, oder, falls $R_1'$ Methyl oder Äthyl bedeutet, auch für den entsprechenden $R_1'-SO_4$-Rest steht, in Gegenwart eines inerten Lösungsmittels umgesetzt werden. Falls als Alkylierungsmittel ein Halogenid, insbesondere Chlorid, der Formel XIII verwendet wird, wird zweckmäßigerweise ein Alkalimetallsalz der Formel IIb eingesetzt, bzw. eine so starke Base verwendet, welche in der Lage ist, in situ ein Alkalimetallsalz mit der Verbindung der Formel IIb zu bilden. Als derartige Basen sind beispielsweise Alkalimetallhydride oder Alkalimetallalkoholate geeignet. Falls ein Dialkylsulfat als Alkylierungsmittel verwendet wird, können beliebige anorganische Basen, beispielsweise Alkalimetallcarbonate oder -hydroxyde wie z. B. Kaliumcarbonat oder Kaliumhydroxyd, verwendet werden. Als inerte Lösungsmittel eignen sich aromatische Kohlenwasserstoffe, niedere Alkohole, Dimethylformamid oder bei Verwendung anorganischer Basen auch niedere Ketone wie Aceton. Gewünschtenfalls können die bei der Cyclisierung entstehenden Alkalimetallsalze der Verbindung der Formel IIb auch ohne vorherige Isolierung der Verbindung der Formel IIb direkt in der Verätherung eingesetzt werden.

Als Hydroxyschutzgruppen eignen sich hydrolytisch oder hydrogenolytisch abspaltbare Gruppen, z. B. Benzyl oder niedere Acylgruppen.

Die für die Verfahrensvariante a') benötigten Ausgangsverbindungen der Formel X können auf an sich bekannte Weise erhalten werden ausgehend von Phenylglycinverbindungen der Formel XIV

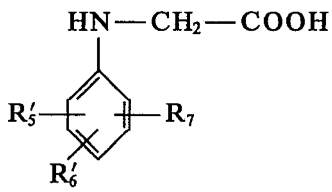

(XIV)

worin $R_5'$, $R_6'$ und $R_7$ obige Bedeutung besitzen, und welche durch Umsetzung der entsprechenden Aniline mit Chloressigsäure erhalten werden können, und

o-Chlorbenzoesäuren der Formel XV

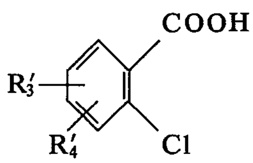

(XV)

worin $R_3'$ und $R_4'$ obige Bedeutung besitzen. Die Alkalimetallsalze, insbesondere Kaliumsalze, der Säuren der Formel XIV werden mit Alkalimetallsalzen, insbesonderem Kaliumsalzen, der Säuren der Formel XV bei erhöhter Temperatur, beispielsweise Temperaturen zwischen 100 und 150°C, in Gegenwart einer anorganischen Base, beispielsweise Kaliumcarbonat, und eines Kupferkatalysators, beispielsweise Kupferpulver, in einem polaren Lösungsmittel, vorzugsweise Wasser oder einer Mischung aus Wasser und einem mit Wasser mischbaren organischen Lösungsmittel, zu Verbindungen der Formel XVI

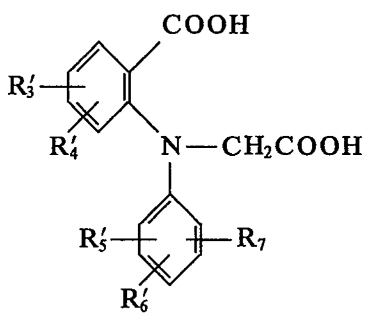

(XVI)

worin $R_3'$, $R_4'$, $R_5'$, $R_6'$ und $R_7$ obige Bedeutung besitzen, umgesetzt, und diese dann in an sich bekannter Weise zu Verbindungen der Formel X verestert, beispielsweise durch Umsetzung mit einem Alkohol $R_{11}OH$ in Gegenwart von Schwefelsäure bei erhöhter Temperatur, beispielsweise Siedetemperatur des Reaktionsgemisches.

Die vorstehend beschriebene Verfahrensvariante a') ist insbesondere geeignet zur Herstellung solcher Verbindungen der Formel IIa, worin $R_3$ und $R_4$ für Wasserstoff oder für solche Substituenten stehen, welche nicht zu einer Umsetzung mit Phenylglycin befähigt sind. Falls die Verbindung der Formel XV weitere zur Umsetzung mit Phenylglycin befähigte Substituenten enthält, können bei dieser Umsetzung neben der Verbindung der Formel XVI auch mehrfach substituierte Nebenprodukte in dem Reaktionsgemisch entstehen. Das gewünschte Reaktionsprodukt kann auf chromatographischem Wege von allfälligen Nebenprodukten abgetrennt werden.

Die Umsetzung von Verbindungen der Formel XI mit Verbindungen der Formel XII gemäß Verfahrensvariante b') kann auf an sich bekannte Weise erfolgen. Zweckmäßigerweise werden die Verbindungen der Formel XI in Form ihrer Alkalimetallsalze, beispielsweise ihrer Natrium- oder Lithiumsalze, mit Verbindungen der Formel XII in einem unter den Reaktionsbedingungen inerten Lösungsmittel bei Temperaturen zwischen ca. 100 und 170°C umgesetzt. Als Lösungsmittel eignen sich in dem angegebenen Temperaturbereich siedende inerte organische Lösungsmittel, vorzugsweise Dimethylformamid. Es ist zweckmäßig, die Reaktion in Gegenwart eines Kupferkatalysators, beispielsweise Kupferpulver, Kupfer-I- oder Kupfer-II-Halogeniden, durchzuführen. Die Alkalimetallsalze der Verbindungen der Formel XI können auf an sich bekannte Weise durch Umsetzen mit einer starken Base, beispielsweise einem Alkalimetallalkoholat oder -hydrid oder -hydroxid, gewünschtenfalls in situ hergestellt werden. Als niedere Acylschutzgruppe zur Herstellung von 3-Hydroxyindol-Verbindungen der Formel IIa sind Acylgruppen mit 2 bis 5 Kohlenstoffatomen, vorzugsweise Acetyl, geeignet.

Die für die Verfahrensvariante b') benötigten Ausgangsverbindungen der Formel XI können auf an sich bekannte Weise ausgehend von Anthranilsäureestern der Formel XVII

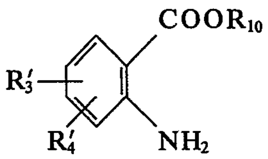

(XVII)

worin $R_3'$ und $R_4'$ obige Bedeutung besitzen und $R_{10}$ niederes Alkyl bedeutet, hergestellt werden, indem man diese zunächst mit Chloressigsäure oder einem Chloressigsäureniederalkylester in an sich bekannter Weise zu Verbindungen der Formel XVIII

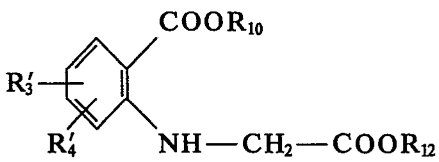

(XVIII)

worin $R_3'$, $R_4'$ und $R_{10}$ obige Bedeutung besitzen und $R_{12}$ Wasserstoff oder niederes Alkyl bedeutet, umsetzt, falls $R_{12}$ Wasserstoff ist, diese Säuren in an sich bekannter Weise mit einem Alkohol $R_{11}OH$, worin $R_{11}$ obige Bedeutung besitzt, verestert, und den erhaltenen Diester in an sich bekannter Weise zu Verbindungen der Formel XIX

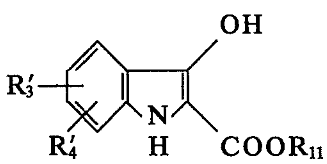

(XIX)

worin $R_3'$, $R_4'$ und $R_{11}$ obige Bedeutung besitzen, cyclisiert, welche dann durch Verätherung bzw. Veresterung der Hydroxygruppe auf an sich bekannte Weise in Verbindungen der Formel XI überführt werden. Die Cyclisierung der Ester der Formel XVIII kann beispielsweise unter den für die Cyclisierung der Ester der Formel X vorstehend beschriebenen Bedingungen erfolgen. Die Verätherung der Verbindungen der Formel XIX kann beispielsweise unter den für die Verätherung der Verbindungen der Formel IIb vorstehend beschriebenen Bedingungen erfolgen.

Die Verfahrensvariante b') eignet sich insbesondere zur Herstellung solcher Verbindungen der Formel IIa, worin $R_5$ bis $R_7$ für Wasserstoff oder für solche Substituenten stehen, welche nicht zu einer Umsetzung mit den Alkalimetallsalzen der Verbindungen der Formel XI befähigt sind.

Verbindungen der Formel III stellen neue wertvolle Zwischenprodukte für die Herstellung von pharmakologisch aktiven Verbindungen, beispielsweise den Verbindungen der Formel I, dar.

Verbindungen der Formel III können auf an sich bekannte Weise erhalten werden, indem man Verbindungen der Formel II auf an sich bekannte Weise mit Verbindungen der Formel XX

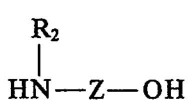

(XX)

worin $R_2$ und Z obige Bedeutung besitzen, umsetzt zu Verbindungen der Formel XXI

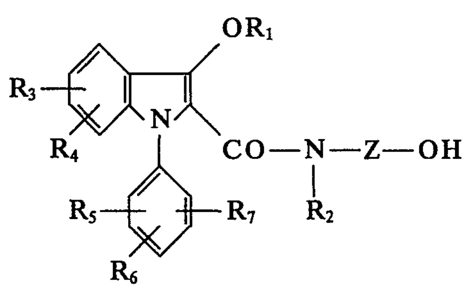

(XXI)

worin $R_1$, $R_2$, $R_3$, $R_4$, $R_5$, $R_6$, $R_7$ und Z obige Bedeutung besitzen, und anschließend die Hydroxygruppe

auf an sich bekannte Weise in eine aminolytisch abspaltbare Gruppe Y überführt.

Die Umsetzung der Verbindungen der Formel II mit den Verbindungen der Formel XX kann unter zur Amidbildung üblichen Bedingungen, beispielsweise den vorstehend für die Umsetzung von Verbindungen der Formel I mit Verbindungen der Formel VI beschriebenen Bedingungen erfolgen. Bei der Umsetzung mit den Verbindungen der Formel II findet überwiegend die gewünschte Amidbildung statt. Die endständige Hydroxygruppe in den Verbindungen der Formel XXI kann auf an sich bekannte Weise in eine aminolytisch abspaltbare Gruppe Y überführt werden, beispielsweise mit gebräuchlichen Halogenierungsmitteln, wie z. B. Thionylchlorid, Phosphoroxychlorid oder Phosphortribromid umgesetzt werden, um Verbindungen der Formel III zu erhalten, worin Y Halogen bedeutet; oder die Hydroxygruppe kann nach an sich bekannten Methoden verestert werden, beispielsweise mit einem entsprechenden Säurehalogenid umgesetzt werden, um Verbindungen der Formel III zu erhalten, worin Y einen reaktiven Esterrest, insbesondere einen der vorgenannten Sulfonsäurereste bedeutet. Bei diesen Umsetzungen reagiert die endständige Hydroxygruppe bevorzugt vor einer allfällig in der Gruppe Z enthaltenen sekundären Hydroxygruppe.

Verbindungen der Formel IVa können erhalten werden, indem man Verbindungen der Formel II in an sich bekannter Weise mit einem Amin der Formel XXII

$$NH_2—R_2 \qquad (XXII)$$

worin $R_2$ obige Bedeutung besitzt, umsetzt. Die Umsetzung kann nach zur Amidbildung üblichen Methoden erfolgen, beispielsweise unter den vorstehend für die Umsetzung von Verbindungen der Formel II mit Verbindungen der Formel VI beschriebenen Bedingungen.

Verbindungen der Formel IVb

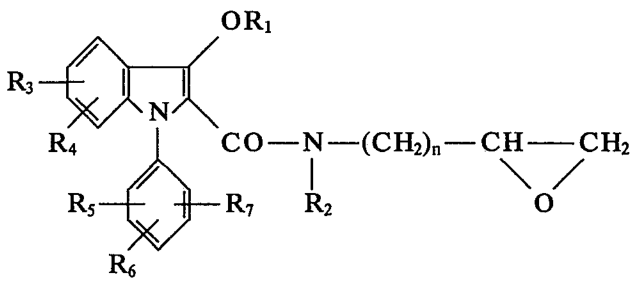

(IVb)

worin $R_2$, $R_3$, $R_4$, $R_5$, $R_6$, $R_7$ und n obige Bedeutung besitzen, können hergestellt werden, indem man Verbindungen der Formel IVa auf an sich bekannte Weise mit Verbindungen der Formel XXIII

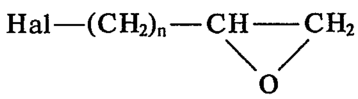

(XXIII)

worin n und Hal obige Bedeutung besitzen, umsetzt. Die Umsetzung kann beispielsweise unter den vorstehend zur Umsetzung von Verbindungen der Formel IVa mit Verbindungen der Formel XIII beschriebenen Bedingungen erfolgen.

Gewünschtenfalls können in den Verbindungen der Formel I oder in den vorgenannten Zwischenprodukten einige der Substituenten in den Phenylringen, wie zum Beispiel Halogen, auf an sich bekannte Weise nachträglich eingeführt werden. So erhält man mit Halogenierungsmitteln wie Chlor, Brom, N-Chlorsuccinimid, N-Chloracetamid oder N-Bromsuccinimid die entsprechenden halogenierten Verbindungen.

Die Verbindungen der Formel I und ihre pharmakologisch akzeptablen Säureadditionssalze zeichnen sich durch interessante pharmakologische Eigenschaften aus und zeigen insbesondere herzrhythmisierende Wirkungen. Die neuen Verbindungen zeichnen sich aus durch eine gute Wirksamkeit und hohe physiologische Verträglichkeit. So zeigen die neuen Verbindungen schon in geringen Dosen eine befriedigende antiarrhythmische Wirkung. Darüber hinaus ist eine unerwünschte negative Beeinflussung des Kontraktionsvermögens des Herzens äußerst gering. Das heißt, die Verbindungen weisen ein besonders günstiges Verhältnis von antiarrhythmischen, bzw. die Refraktärzeit des Herzens verlängernde Wirkungen zu negativ inotropen Nebenwirkungen auf und besitzen eine große therapeutische Breite.

Die antiarrhythmischen Wirkungen der Verbindungen lassen sich in pharmakologischen Standardtestmethoden nachweisen.

Zum Beispiel zeigen die Verbindungen in Mäusen eine hemmende Wirkung auf durch Chloroforminhalation hervorgerufenes Kammerflimmern. Die Wirkung der Verbindungen auf die durch zu schnellem Atemstillstand führende Chloroforminhalation hervorgerufene ventrikuläre Fibrillation in Mäusen wird nach der Methode nach Lawson (J. Pharmacol. Exp. Ther. 160, 22—23) bestimmt.

In dieser Versuchsanordnung kann gleichzeitig auch die minimale toxische Dosis bestimmt werden. Die Testsubstanzen werden weiblichen Mäusen von 17—24 g Körpergewicht in 0,9%iger 30 NaCl-

Lösung gelöst i. p. verabreicht. Die Tiere werden einzeln in Glasbechern gehalten, wo sie auf mögliche toxische Symptome beobachtet werden. 10 Minuten nach Verabreichung der Testsubstanzen werden die Tiere in bedeckte 300 ml Glasbecher umgesetzt, welche einen mit ungefähr 20 ml Chloroform getränkten Wattebausch enthalten. Sofort nach Eintreten des Atemstillstands wird das Herz freigelegt und ventrikularer Rhythmus und Geschwindigkeit werden visuell beobachtet. Es wird angegeben, welcher Prozentsatz der Tiere durch die verabreichte Dosis gegen das Kammerflimmern geschützt wird.

Die Verbindungen der Formel I zeigen in dem vorstehend beschriebenen Test in einem Dosisbereich von 0,1—100 mg/kg antiarrhythmische Wirkungen.

Die folgende Tabelle gibt nach der vorstehend beschriebenen Testmethode erhaltene Ergebnisse wieder. Außerdem sind in der Tabelle die minimalen toxischen Dosen nach intraperitonalen (i.p.) und (p.o.) Verabreichung angegeben. Die für die Verbindungen der Formel I angegebenen Beispielsnummern beziehen sich auf die nachstehenden Herstellungsbeispiele.

| Testsubstanz der Formel I Beispiel-Nr. | Antiarrhythmische Wirkung | | Minimale toxische Dosis mg/kg | |
|---|---|---|---|---|
| | Dosis mg/kg ip | % geschützte Tiere | ip | po |
| 25 | 100 | 66,7 | ≧100 | ≧300 |
| 2 | 100 | 100 | ≧100 | ≧300 |
| 15 | 100 | 100 | ≧100 | ≧300 |
| 20 | 10 | 100 | 50 | 300 |
| 1 | 25 | 100 | 100 | 300 |
| 22 | 10 | 66,7 | 50 | 300 |
| 30 | 50 | 33,3 | 200 | ≧300 |

Ebenso zeigen die Verbindungen in Ratten in der Versuchsmethode nach Raschak (Arzneimittelforsch. 25 [1975] 639—641) eine hemmende Wirkung gegenüber den durch Aconitin-Infusion induzierten Herzrhythmusstörungen [Extrasystolen (ES), ventrikulärer Tachycardie (VT) Kammerflattern (KF)]. So sind z. B. die minimalen effektiven Dosen von 2-[3-(N,N-Diäthylamino)-2-hydroxypropylaminocarbonyl]-3-methoxy-1-phenylindol bei iv Applikation 5 Minuten vor Beginn der Aconitininfusion wie folgt:

| Effekt | Minimale effektive hemmende Dosis µmol/kg |
|---|---|
| Extrasystolen | 2,15 |
| Ventriculäre Tachycardie | 2,15 |
| Kammerflattern | 4,64 |
| Herzfrequenz | >12 |

Dabei zeigt die Substanz bei der Prüfung der akuten Toxicität eine $LD_{50}$ von 164 µmol/kg ip und von 37,3 µmol/kg iv. Daraus ergibt sich eine therapeutische Breite (= Quotient $LD_{50\,Maus}/ED_{min}$) von 8—17.

Das günstige Verhältnis von die funktionelle Refraktärzeit verlängernder Wirkung (FRZ) zu die Kontraktionskraft vermindernder (Kraft) der Verbindungen läßt sich auch am isolierten linken Vorhof von weiblichen Meerschweinchen von 300—400 g Körpergewicht gemäß der Doppelreizmethode von Govier [J. Pharmakol. Exp. Ther. 148 [1965] 100—105] zeigen. In der nachfolgenden Tabelle sind angegeben diejenige Konzentration in µmol/l, bei der es 18 Minuten nach Applikation zu einer Verlängerung der funktionellen Refraktärzeit auf 125% kommt und diejenige Konzentration, bei der es zu einer Verminderung der Kontraktionskraft auf 75% des Ausgangswertes kommt, und außerdem der Quotient aus diesen Konzentrationen, welcher ein Indiz für die therapeutische Breite der Verbindungen darstellt.

| Beispiel Nr. Nr. | Isolierter Meerschweinchen-Vorhof | | |
|---|---|---|---|
| | Kraft [μmol/l] | FRZ [μmol/l] | Kraft FRZ |
| 20 | 3,57 | 0,66 | 5,4 |
| 1 | 6,3 | 3,6 | 1,8 |

Aufgrund der vorstehend beschriebenen Wirkungen eignen sich die Verbindungen der Formel I und ihre pharmakologisch akzeptablen Säureadditionssalze als Arzneimittel zur Behandlung von Herzrhythmusstörungen. Ferner zeigen die Substanzen der Formel I auch antithrombotische Eigenschaften. Als Heilmittel können die Verbindungen der Formel I und ihre physiologisch verträglichen Säureadditionssalze zusammen mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z. B. Milchzucker, Stärke oder Talkum oder flüssiger Verdünnungsmittel wie z. B. Wasser, fetten Ölen oder flüssigen Paraffinen.

Die nachfolgenden Beispiele sollen die Herstellung der neuen Verbindungen der Formel I sowie der neuen Zwischenprodukte näher erläutern jedoch den Umfang der Erfindung in keiner Weise beschränken.

### Beispiel 1

2-[3-(N,N-Diäthylamino)-2-hydroxypropylaminocarbonyl]-3-methoxy-1-phenylindol

A) 475 g Chloressigsäure (5 Mol) werden mit 930 g Anilin (10 Mol) in 2 l Wasser 1,5 Stunden lang auf 100°C erhitzt. Nach dem Abkühlen wird das gebildete N-Phenylglycin abgesaugt und mit Wasser gewaschen. Ausbeute 468 g (= 62% bezogen auf Chloressigsäure).

B) 468 g N-Phenylglycin werden unter Erwärmung in 1,3 l Methanol gelöst. Die Lösung wird unter Kühlung mit einer Lösung von 205 g Kaliumhydroxyd in 450 ml Methanol versetzt. Nach dem Abkühlen wird das ausgefallene Kaliumsalz des N-Phenylglycins abgesaugt. Ausbeute 392 g = 67%.

C) 468 g o-Chlorbenzoesäure werden unter Erwärmung in 1,5 l Isopropanol gelöst. Die Lösung wird unter Kühlung mit einer Lösung von 198 g Kaliumhydroxyd in 200 ml Methanol versetzt. Nach dem Abkühlen wird das ausgefallene Kaliumsalz der o-Chlorbenzoesäure abgesaugt. Ausbeute 394 g = 67,4%.

D) 750 g des Kaliumsalzes des N-Phenylglycins werden mit 808 g des Kaliumsalzes der o-Chlorbenzoesäure, 268 g Kaliumcarbonat und 1,5 g Kupfer-Pulver in 385 ml Wasser 5 Stunden lang auf 120 bis 125°C (Innentemperatur) erhitzt. Nach dem Lösen des Reaktionsgemisches in Wasser wird die Lösung mit Salzsäure angesäuert und die ausgefallene N-Diphenylglycin-o-carbonsäure abgesaugt. Ausbeute 675 g = 62,7%.

E) 675 g N-Diphenylglycin-o-carbonsäure werden mit 2,5 l Methanol und 500 ml Schwefelsäure 5 Stunden lang zum Sieden erhitzt. Das Methanol wird teilweise abgedampft, die Reaktionsmischung anschließend in Wasser gegossen und mit Methylenchlorid extrahiert. Die Methylenchloridphase wird mit Sodalösung ausgeschüttelt, getrocknet und eingedampft, wobei der rohe N-Diphenylglycin-o-carbonsäuredimethylester als Rückstand erhalten wird. Ausbeute 614 g Rohprodukt = 82,4%.

F) 47,1 g Natrium werden in 500 ml Methanol gelöst und die Lösung mit 500 ml Toluol versetzt. Das Gemisch wird zum Sieden erhitzt und unter schwachem Rückfluß mit einer Lösung von 614 g N-Diphenylglycin-o-carbonsäuredimethylester in 1,5 l Toluol versetzt. Nach weiteren 30 Minuten Siedens wird die Reaktionsmischung abgekühlt und in 1 l Wasser gegeben und mit 250 ml Salzsäure angesäuert. Der ausgefallene N-Phenylindoxylsäuremethylester wird abgesaugt. Ausbeute 464 g = 84,8%.

G) 140 g N-Phenylindoxylsäuremethylester werden in 600 ml Aceton mit 69 ml Dimethylsulfat und 71 g Kaliumcarbonat während 4 Stunden unter Rühren zum Sieden erhitzt. Die Reaktionsmischung wird in Wasser gegossen und der gebildete N-Phenyl-3-methoxyindol-2-carbonsäuremethylester wird abgesaugt und in 450 ml Methanol gelöst. Die Lösung wird mit einer Lösung von 42 g Natriumhydroxyd in 50 ml Wasser versetzt und 30 Minuten lang zum Sieden erhitzt. Das Reaktionsgemisch wird in Wasser gelöst, die wäßrige Lösung wird mit Salzsäure angesäuert und

die ausgefallene N-Phenyl-3-methoxyindol-2-carbonsäure wird abgesaugt. Ausbeute 127 g = 90,7%.

H) 92 g N-Phenyl-3-methoxyindol-2-carbonsäure werden in 920 ml Äther und 30,3 g Pyridin gelöst. Die Lösung wird unter Eiskühlung in eine Lösung von 28,3 ml Thionylchlorid in 160 ml Äther unter Rühren eingetropft. Die Mischung wird 1 Stunde bei Raumtemperatur gerührt und die ausgefallenen Pyridinsalze werden abgesaugt.
Die erhaltene ätherische Lösung des N-Phenyl-3-methoxyindol-2-carbonsäurechlorids wird unter Eiskühlung zu einer Lösung von 579 g 1-Amino-2-hydroxy-3-diäthylaminopropan und 40 g Triäthylamin in 70 ml Methylenchlorid getropft. Das Reaktionsgemisch wird 1 Stunde bei Raumtemperatur gerührt und anschließend mit verdünnter Salzsäure extrahiert. Der Salzsäureextrakt wird durch Zugeben von Natronlauge alkalisch gemacht und mit Äther extrahiert. Die Ätherlösung wird mit Wasser gewaschen, über Natriumsulfat getrocknet und eingedampft. Es verbleiben 110 g 2-[3-(N,N-Diäthylamino)-2-hydroxypropylaminocarbonyl]-3-methoxy-1-phenylindol als ölige Base. Diese wird in Isopropanol gelöst und in ihr Hydrochlorid überführt. Ausbeute 110 g des Hydrochlorids = 60,6%, Schmelzpunkt 148—150°C.

## Beispiel 2

### 2-[2-(N,N-Dimethylamino)-äthylaminocarbonyl]-3-hydroxy-1-(4'-chlorphenyl)-indol

16,3 g N-(4'-Chlorphenyl)-indoxylsäuremethylester werden mit 30 ml 1-Dimethylamino-2-aminoäthan 2 Stunden lang zum Sieden erhitzt. Das überschüssige Amin wird abdestilliert und die als Rückstand verbleibende rohe Titelverbindung wird in verdünnter wäßriger Salzsäure gelöst. Das Hydrochlorid der Titelverbindung fällt aus und wird abgesaugt. Ausbeute 14,1 g Rohprodukt. Das rohe Hydrochlorid wird dreimal aus Methanol/Äther umgefällt und einmal aus Isopropanol umkristalliert. Ausbeute 10,0 g, Schmelzpunkt 188 bis 189°C (Zersetzung).

## Beispiel 3

### 2-[3-(N,N-Diäthylamino)-2-hydroxy-propylaminocarbonyl]-3-äthoxy-1-phenylindol

A) 100 g N-Phenylindoxylsäuremethylester (hergestellt analog Beispiel 1 F) und 16,8 g Natriumhydroxyd werden in 400 ml Methanol gelöst. Die Lösung wird eingedampft und der Rückstand in 300 ml Dimethylformamid gelöst. 32 ml Äthyljodid werden portionsweise zu dieser Lösung gegeben und die Reaktionsmischung wird 5 Stunden bei 120°C gehalten. Danach wird das Lösungsmittel eingedampft, der Rückstand mit Cyclohexan ausgerührt und abgesaugt. Die Lösung wird eingedampft. Es werden 77,35 g 3-Äthoxy-1-phenylindol-2-carbonsäure-methylester als öliges Rohprodukt erhalten. Dieses kann chromatografisch gereinigt werden oder direkt in die nachfolgende Reaktion eingesetzt werden.

B) 77,35 g 3-Äthoxy-1-phenylindol-2-carbonsäure-methylester werden mit 200 ml 50%igem Äthanol und 10,5 g Natriumhydroxyd während 3 Stunden unter Rückfluß gekocht. Anschließend wird das Lösungsmittel abgedampft, der Rückstand mit Eis zersetzt und die Mischung mit verdünnter Salzsäure angesäuert. Die ausgefallene rohe 3-Äthoxy-1-phenylindol-2-carbonsäure wird abgesaugt. Das Rohprodukt wird in Dichlormethan gelöst, die Lösung mit Natriumsulfat getrocknet, eingedampft und der Rückstand aus Äther/Petroläther umkristallisiert. Ausbeute 25,4 g.

C) 1,42 g 3-Äthoxy-1-phenylindol-2-carbonsäure werden in 10 ml Dichlormethan mit 0,85 ml Oxalylchlorid versetzt und die Mischung 2 Stunden bei Raumtemperatur gerührt. Die Mischung wird zur Trocknung eingedampft und in Dioxan aufgenommen. Die Dioxanlösung wird wiederum eingedampft, der Rückstand in Dioxan gelöst und die Lösung mit 1 ml 3-(N,N-Diäthylamino)-2-hydroxypropylamin versetzt. Nach Beendigung der Reaktion wird die Lösung eingedampft, der Rückstand in Äther aufgenommen, die Lösung mit gesättigter Kochsalz-Lösung gewaschen, die organische Phase getrocknet und eingedampft. Das als Rückstand verbleibende 2-[3-(N,N-Diäthylamino)-2-hydroxy-propylaminocarbonyl]-3-äthoxy-1-phenylindol wird in Isopropanol/Äther gelöst, die Lösung mit gasförmigen Chlorwasserstoff versetzt und das auskristallisierende Hydrochlorid wird abgesaugt. Schmelzpunkt 146—148°C.

## Beispiel 4

### 5-Brom-2-[3-(N,N-Diäthylamino)-2-hydroxy-propylaminocarbonyl]-3-methoxy-1-phenylindol

A) 100 g 3-Brom-6-chlorbenzoesäure werden in 900 ml Isopropanol gelöst. Die Lösung wird mit 23,6 g Kaliumhydroxyd in 225 ml Methanol versetzt. Unter Rühren wird abgekühlt das ausgefallene Kaliumsalz der 3-Brom-6-chlorbenzoesäure wird abgesaugt und getrocknet. Ausbeute 103 g.

B) 103 g des Kaliumsalzes der 3-Brom-6-chlorbenzoesäure werden mit 68,2 g des Kaliumsalzes des Phenylglycins, 25,5 g Kaliumcarbonat, 0,5 g Kupferpulver und 90 ml Wasser 4 Stunden lang auf 120°C erhitzt. Das Reaktionsgemisch wird mit Wasser verdünnt, mit konzentrierter Salzsäure angesäuert, und mit Dichlormethan extrahiert. Die organische Phase wird über Natriumsulfat getrocknet, filtriert und eingedampft, wobei das N-(4-Brom-2-hydroxycarbonylphenyl)-N-phenylglyzin als öliger Rückstand erhalten wird. Ausbeute 84,7 g.

C) 84,7 g der vorstehenden Säure werden in 250 ml Methanol gelöst. Unter Rühren werden 64 ml Schwefelsäure in die Lösung eingetropft und das Reaktionsgemisch 4 Stunden bei Rückfluß gekocht. Anschließend wird das Methanol abgedampft, das verbleibende Öl in Wasser gerührt und mit Dichlormethan extrahiert. Die organische Phase wird abgetrennt, getrocknet und eingedampft. Der als Öl zurückbleibende Dimethylester des N-(4-Brom-2-hydroxycarbonylphenyl)-N-phenylglycin wird am Kugelrohr destilliert. Ausbeute 31 g Öl $Kp_{0,1}$ 120—140°C.

D) 2,1 g Natrium werden in 30 ml Methanol gelöst, 30 ml Toluol zu der Lösung hinzugefügt und zum Sieden erhitzt. Unter Sieden bei Rückfluß wird eine Lösung von 31,1 g des vorstehenden Diesters in 75 ml Toluol zugetropft. Die Mischung wird eine weitere Stunde unter Rückfluß gekocht. Anschließend wird abgekühlt und mit verdünnter Salzsäure angesäuert. Der ausgefallene 5-Brom-1-phenyl-indoxylsäuremethylester wird abgesaugt. Ausbeute 12 g.

E) 12 g 5-Brom-1-phenylindoxylsäuremethylester werden mit 4,7 g Kaliumcarbonat in 60 ml Aceton suspendiert. Zu dieser Mischung gibt man 2,5 ml Dimethylsulfat und kocht 2 Stunden unter Rückfluß. Nach dem Abkühlen wird mit Wasser verdünnt und der ausgefallene 5-Brom-3-methoxy-1-phenylindol-2-carbonsäuremethylester wird abgesaugt. Ausbeute 12,9 g.

F) 12,5 g des vorstehenden Esters werden in 100 ml Äthanol (50%) gelöst und die Lösung mit 1,6 g Natriumhydroxyd 2 Stunden unter Rückfluß gekocht. Das Äthanol wird abgedampft und die verbleibende wäßrige Lösung wird mit verdünnter Salzsäure angesäuert und mit Dichlormethan extrahiert. Die organische Phase wird über Natriumsulfat getrocknet, filtriert und eingedampft. Die verbleibende rohe 5-Brom-3-methoxy-1-phenylindol-2-carbonsäure wird aus Äther/Petroläther umkristallisiert. Schmelzpunkt 169—173°C, Ausbeute 7,5 g.

G) 1,75 g der vorstehenden Säure werden in 100 ml Dichlormethan mit 0,35 ml Oxalylchlorid versetzt und die Mischung 2 Stunden bei Raumtemperatur gerührt. Dann wird zur Trocknung eingedampft und der Rückstand in Dioxan aufgenommen, nochmals eingedampft und in Dioxan gelöst. Die Lösung wird mit 1 ml 3-(N,N-Diäthylamino)-2-hydroxypropylamin tropfenweise versetzt. Nach Beendigung der Reaktion wird eingedampft, der Rückstand in Äther aufgenommen, mit gesättigter Sodalösung gewaschen, die organische Phase abgetrennt, getrocknet und eingedampft. Das verbleibende 5-Brom-2-[3-(N,N-diäthylamino)-2-hydroxy-propylaminocarbonyl]-3-methoxy-1-phenylindol wird in Isopropanol/Äther gelöst und die Lösung mit gasförmigem Chlorwasserstoff versetzt, wobei das Hydrochlorid der Titelverbindung auskristallisiert. Schmelzpunkt 201—203°C.

## Beispiel 5

### 5-Brom-2-[3-(N,N-diäthylamino)-2-hydroxy-propylaminocarbonyl]-3-methoxy-1-phenylindol

16 g 3-Methoxy-1-phenylindol-2-carbonsäuremethylester (hergestellt analog Beispiel 1 G) werden in 250 ml Eisessig gelöst und zu der Lösung werden 1,6 ml Brom tropfenweise zugesetzt. Die Lösung wird 3 Stunden bei 100°C gehalten, dann abgekühlt und auf Eis gegossen. Die Mischung wird mit Dichlormethan extrahiert, die organische Phase getrocknet, eingedampft, und der zurückbleibende 5-Brom-3-methoxy-1-phenylindol-2-carbonsäuremethylester wird aus Äther kristallisiert.

Der 5-Brom-3-methoxy-1-phenylindol-2-carbonsäuremethylester wird anschließend analog Beispiel 4F und G weiterverarbeitet, wobei das Hydrochlorid der Titelverbindung erhalten wird. Schmelzpunkt 201—203°C.

## Beispiel 6

### 5-Methyl-2-[3-(N,N-diäthylamino)-2-hydroxy-propylaminocarbonyl]-3-methoxy-1-phenylindol

A) 12 g 3-Methoxy-5-methylindol-2-carbonsäuremethylester werden in 25 ml Dimethylformamid gelöst. Zu dieser Lösung werden 1,5 g Natriumhydroxid (80%ig) portionsweise zugefügt. Nach beendeter Reaktion werden 10 g Kupferjodid zugefügt und die Reaktionsmischung eine halbe Stunde lang auf 100°C erhitzt. Anschließend werden 8 ml Brombenzol zugefügt und die Mischung unter Rühren während 20 Stunden auf 140°C erhitzt. Nach Abkühlen und Zugabe von Wasser und Methylenchlorid wird die ausgefallene anorganische Substanz abfiltriert, die Methylenchloridphase abgetrennt, getrocknet und eingedampft. Der zurückbleibende 3-Methoxy-5-methyl-1-phenyl-indol-2-carbonsäuremethylester wird mit 3 g Natriumhydroxyd, 3 ml Wasser und 30 ml Methanol 30 Minuten lang zum Sieden

erhitzt. Anschließend wird das Lösungsmittel abgedampft, der Rückstand in Wasser gelöst, die wäßrige Lösung mit Äther extrahiert und dann mit Salzsäure auf pH 3 angesäuert. Die ausgefallene 3-Methoxy-5-methyl-1-phenyl-indol-2-carbonsäure wird abgesaugt und aus Cyclohexan umkristallisiert. Schmelzpunkt 121—122 °C, Ausbeute 9,6 g.

B) Die vorstehende 3-Methoxy-5-methyl-1-phenyl-indol-2-carbonsäure wird analog Beispiel 1 H in ihr Säurechlorid überführt und dieses mit 1-Amino-3-diäthylamino-2-hydroxypropan umgesetzt. Die Titelverbindung wird als ölige Base erhalten. Diese wird in Isopropanol gelöst, die Lösung mit Zitronensäure versetzt. Das ausgefallene Citrat der Titelverbindung wird abgesaugt und aus Isopropanol umkristallisiert. Schmelzpunkt 126—128 °C, Ausbeute 7,7 g.

Beispiel 7

2-[3-(N,N-Diäthylamino)-2-hydroxypropylaminocarbonyl]-3-methoxy-1-(2',4'-dimethoxyphenyl)-indol

16,8 g 3-Methoxy-1-(2',4'-dimethoxyphenyl)-indol-2-carbonsäure und 7 ml Triäthylamin werden in 50 ml Dichlormethan gelöst und zu 4 ml Thionylchlorid in 5 ml Diochlormethan getropft. In diese Lösung werden 8 g 3-(N,N-Diäthylamino)-2-hydroxypropylamin gelöst in 10 ml Dichlormethan eingetropft. Die Lösung wird in Eiswasser gegossen, mit Dichlormethan extrahiert und die organische Phase mit Wasser gewaschen, getrocknet und eingedampft. Das verbleibende 2-[3-(N,N-Diäthylamino)-2-hydroxy-propylaminocarbonyl]-3-methoxy-1-(2',4'-dimethoxyphenyl)-indol wird in Isopropanol gelöst und die Lösung mit gasförmigem Chlorwasserstoff versetzt, wobei das Hydrochlorid der Titelverbindung auskristallisiert. Ausbeute 13,4 g, Schmelzpunkt 166—168 °C.

Beispiel 8

2-[3-(N,N-Diäthylamino)-2-hydroxypropylaminocarbonyl]-3-methoxy-1-phenylindol

Zu 13,3 g N-Phenyl-3-methoxyindol-2-carbonsäure gelöst in 100 ml Dichlormethan werden 15 g 2-Chlor-N-methylpyridiniumjodid in 150 ml Dichlormethan und 14 ml Triäthylamin gegeben und eine Stunde bei Raumtemperatur gerührt. Dann werden 7,5 g 3-(N,N-Diäthylamino)-2-hydroxypropylamin zugefügt und es wird weitere 3 Stunden bei Raumtemperatur gerührt. Die Reaktionslösung wird in Wasser gegeben und mit Dichlormethan extrahiert. Die organische Phase wird mit verdünnter Natronlauge und Wasser gewaschen, getrocknet und eingedampft. Die erhaltene ölige Titelverbindung wird entsprechend Beispiel 1 in ihr Hydrochlorid überführt. Schmelzpunkt 148—150 °C, Ausbeute 12 g.

Beispiel 9

2-[3-(N,N-Diäthylamino)-2-hydroxypropylaminocarbonyl]-3-methoxy-1-phenylindol

6,6 g N-Phenyl-3-methoxyindol-2-carbonsäure und 7 ml Triäthylamin in 75 ml Dichlormethan werden bei —30 °C zu 7 g Chlorameisensäureäthylester in 25 ml Dichlormethan getropft. Unter Rühren läßt man die Temperatur langsam bis auf 5 °C ansteigen und fügt 3,7 g 3-(N,N-Diäthylamino)-2-hydroxypropylamin zu. Es wird bei Raumtemperatur nachgerührt, die Reaktionslösung in Wasser gegossen und wie in Beispiel 8 beschrieben aufgearbeitet. Ausbeute 4,5 g Hydrochlorid der Titelverbindung, Schmelzpunkt 148—150 °C.

Beispiel 10

2-[3-(N,N-Diäthylamino)-2-hydroxypropylaminocarbonyl]-3-methoxy-1-phenylindol

A) Zu 13,3 g N-Phenyl-3-methoxyindol-2-carbonsäure gelöst in 100 ml Dichlormethan werden 15 g 2-Chlor-N-methylpyridiniumjodid in 150 ml Dichlormethan und 14 ml Triäthylamin gegeben. Nach einer Stunde bei Raumtemperatur werden 5,5 g 3-Amino-1,2-propandiol in 50 ml Pyridin zugefügt und die Reaktionslösung über Nacht gerührt. Dann wird eingedampft, mit Dichlormethan aufgenommen und nacheinander mit verdünnter Salzsäure, verdünnter Natronlauge und gesättigter Kochsalzlösung gewaschen. Nach dem Trocknen und Eindampfen der organischen Phase wird das zurückbleibende Öl in Essigsäureäthylester aufgenommen und kristallisiert. Ausbeute: 8,1 g 2-[2,3-Dihydroxypropylaminocarbonyl]-3-methoxy-1-Phenylindol.

B) 1,7 g 2-[2,3-Dihydroxypropylaminocarbonyl]-3-methoxy-1-phenylindol und 1,2 g p-Toluolsulfonsäurechlorid werden in 20 ml trockenem Pyridin 24 Stunden bei Raumtemperatur gerührt und die

Reaktionslösung dann im Vakuum eingedampft, wobei als Rückstand rohes 2-[3-(p-Toluolsulfonyloxy)-2-hydroxypropylaminocarbonyl]-3-methoxy-1-phenylindol erhalten wird.

C) Das vorstehend erhaltene Rohprodukt wird in 30 ml Methanol aufgenommen, mit 3 ml Diäthylamin versetzt und 24 Stunden bei 80°C gerührt. Anschließend wird eingedampft, mit verdünnter Salzsäure aufgenommen und wie in Beispiel 1 H aufgearbeitet und man erhält das Hydrochlorid des 2-[3-(N,N-Diäthylamino)-2-hydroxypropylaminocarbonyl]-3-methoxy-1-phenylindol. Schmelzpunkt 148—150°C, Ausbeute: 0,5 g.

## Beispiel 11

### 2-{N-[3-(N',N'-Diäthylamino)-propyl]-N-äthylaminocarbonyl}-3-methoxy-1-phenylindol

2,9 g 2-Äthylaminocarbonyl-3-methoxy-1-phenylindol (hergestellt aus N-Phenyl-3-methoxyindol-2-carbonsäure und Äthylamin analog Beispiel 8) werden in 35 ml Dimethylformamid gelöst und zur Bildung des Natriumsalzes bei Eiskühlung mit 0,5 g Natriumhydrid (80%) versetzt. Nach 1 Stunde bei Raumtemperatur werden 1,5 g 3-Diäthylaminopropylchlorid in 10 ml Dimethylformamid zugefügt und die Reaktionslösung auf 60°C erwärmt und 2 Stunden bei dieser Temperatur gehalten. Dann wird im Vakuum eingedampft, der Rückstand in verdünnter Salzsäure aufgenommen und mit Äther gewaschen. Die salzsaure Lösung wird mit Sodalösung alkalisch gestellt, mit Äther extrahiert und die organische Phase mit Natriumsulfat getrocknet und eingedampft. Man erhält die Titelverbindung als ölige Base. Ausbeute: 4,0 g.

## Beispiel 12

### 2-{N-[3-(N',N'-Diäthylamino)-2-hydroxypropyl]-N-äthylaminocarbonyl}-3-methoxy-1-phenylindol

A) 2,9 g 2-Äthylaminocarbonyl-3-methoxy-1-phenylindol werden entsprechend Beispiel 11 mit Natriumhydrid umgesetzt. Anschließend werden zu dem Reaktionsgemisch 1,0 g Epichlorhydrin in 10 ml Dimethylformamid zugefügt und die Reaktionslösung während 3 Stunden auf 80°C erwärmt, wobei sich in der Reaktionslösung 2-[N-(2,3-Epoxypropyl)-N-äthylaminocarbonyl]-3-methoxy-1-phenylindol bildet.

B) Zu der vorstehend erhaltenen Reaktionslösung werden 0,9 g Diäthylamin zugesetzt und weitere 3 Stunden erwärmt. Anschließend wird entsprechend Beispiel 11 aufgearbeitet. Man erhält 3,0 g 2-{N-[3-(N',N'-Diäthylamino)-2-hydroxypropyl]-N-äthylaminocarbonyl}-3-methoxy-1-phenylindol als ölige Base.

Nach den in Beispiel 1 bis 12 beschriebenen Verfahren können auch die in der folgenden Tabelle aufgeführten 2-(Aminoalkylaminocarbonyl)-1-phenylindol-Verbindungen der Formel I aus entsprechenden 1-Phenylindol-2-carbonsäurederivaten, bzw. -carbonsäureamidderivaten hergestellt werden.

| Bsp. Nr. | $R_1$ | $R_3$ | $R_4$ | $R_5$ | $R_6$ | $R_7$ | Z | $R_2$ | $R_8$ | $R_9$ | Salz | Fp °C |
|---|---|---|---|---|---|---|---|---|---|---|---|---|
| 13 | $CH_3$ | H | H | 2,3-di—$CH_3$ | | H | $C_2H_4$ | H | $CH_3$ | $CH_3$ | HCl | 223–224 |
| 14 | $CH_3$ | H | H | 2,3-di—$CH_3$ | | H | n-$C_3H_6$ | H | $CH_3$ | $CH_3$ | HCl | 168–170 |
| 15 | $CH_3$ | H | H | 4-Cl | H | H | $C_2H_4$ | H | $CH_3$ | $CH_3$ | HCl | 184–187 |
| 16 | $CH_3$ | H | H | 4-Cl | H | H | n-$C_3H_6$ | H | $CH_3$ | $CH_3$ | HCl | 183–187 |
| 17 | $CH_3$ | H | H | H | H | H | $C_2H_4$ | H | $CH_3$ | $CH_3$ | HCl | 202–204 |
| 18 | $CH_3$ | H | H | H | H | H | $C_2H_4$ | H | $C_2H_5$ | $C_2H_5$ | HCl | 191–192 |
| 19 | $CH_3$ | H | H | H | H | H | n-$C_3H_6$ | H | $CH_3$ | $CH_3$ | HCl | 178–180 |
| 20 | $CH_3$ | H | H | H | H | H | n-$C_3H_6$ | H | $C_2H_5$ | $C_2H_5$ | HCl | 135–137 |
| 21 | $CH_3$ | 4,5-di—Br | | H | H | H | $CH_2$—CHOH—$CH_2$ | H | $C_2H_5$ | $C_2H_5$ | HCl | |
| 22 | $CH_3$ | H | H | H | H | H | n-$C_4H_8$ | H | $C_2H_5$ | $C_2H_5$ | HCl | 115–117 |
| 23 | $C_2H_5$ | H | H | H | H | H | $C_2H_4$ | H | $CH_3$ | $CH_3$ | HCl | 179–181 |
| 24 | H | H | H | H | H | H | $C_2H_4$ | H | $CH_3$ | $CH_3$ | HCl | 188–193 |
| 25 | H | H | H | 2,3-di—$CH_3$ | | H | $C_2H_4$ | H | $CH_3$ | $CH_3$ | HCl | 212–213 |
| 26 | $CH_3$ | H | H | H | H | H | $CH_2$CHOH—$CH_2$ | H | -n—$C_4H_8$— | | HBr | 164–165 |
| 27 | $CH_3$ | H | H | H | H | H | $CH_2$—CHOH—$CH_2$ | H | —n$C_5H_{10}$— | | HBr | 206–207 |
| 28 | $CH_3$ | H | H | H | H | H | $CH_2$—CHOH—$CH_2$ | H | —$C_2H_4OC_2H_4$— | | HCl | 195–196 |
| 29 | $CH_3$ | H | H | H | H | H | $CH_2$—CHOH—$CH_2$ | H | —$C_2H_4SC_2H_4$ | | HBr | 217–218 |

Fortsetzung

| Bsp. Nr. | $R_1$ | $R_3$ | $R_4$ | $R_5$ | $R_6$ | $R_7$ | Z | $R_2$ | $R_8$ | $R_9$ | Salz | Fp °C |
|---|---|---|---|---|---|---|---|---|---|---|---|---|
| 30 | $CH_3$ | 5-Br | H | H | H | H | $C_2H_4$ | H | $CH_3$ | $CH_3$ | HCl | 238–242 |
| 31 | $CH_3$ | 5-Br | H | H | H | H | n-$C_3H_6$ | H | $C_2H_5$ | $C_2H_5$ | HCl | 177–179 |
| 32 | $CH_3$ | 4,5-di—Br | | H | H | H | n-$C_3H_6$ | H | $C_2H_5$ | $C_2H_5$ | HCl | 222–226 |
| 33 | $CH_3$ | 4,5-di—Br | | H | H | H | $C_2H_4$ | H | $CH_3$ | $CH_3$ | HCl | |
| 34 | $C_2H_5$ | H | H | H | H | H | n-$C_3H_6$ | H | $C_2H_5$ | $C_2H_5$ | HCl | 154–256 |
| 35 | n-$C_7H_{15}$ | H | H | H | H | H | $CH_2$—CHOH—$CH_2$ | H | $C_2H_5$ | $C_2H_5$ | Base | Öl |
| 36 | n-$C_3H_7$ | H | H | H | H | H | $CH_2$—CHOH—$CH_2$ | H | $C_2H_5$ | $C_2H_5$ | 120–123 | |
| 37 | $C(CH_3)_3$ | H | H | H | H | H | $CH_2$CHOH—$CH_2$ | H | $C_2H_5$ | $C_2H_5$ | | |
| 38 | $CH_2$—$CH(CH_3)_2$ | H | H | H | H | H | $CH_2$—CHOH—$CH_2$ | H | $C_2H_5$ | $C_2H_5$ | | |
| 39 | $CH_3$ | H | H | 4-$OCH_3$ | H | H | $CH_2$—CHOH—$CH_2$ | H | $C_2H_5$ | $C_2H_5$ | HCl | 204–205 |
| 40 | $CH_3$ | H | H | 3-Br | H | H | $CH_2$—CHOH—$CH_2$ | H | $C_2H_5$ | $C_2H_5$ | HCl | 135–137 |
| 41 | $CH_3$ | 7-$CH_3$ | H | H | H | H | $CH_2$—CHOH—$CH_2$ | H | $C_2H_5$ | $C_2H_5$ | HCl | 125–127 |
| 42 | $CH_3$ | H | H | 2-$CH_3$ | 3-Cl | H | $CH_2$—CHOH—$CH_2$ | H | $C_2H_5$ | $C_2H_5$ | HCl | 219–221 |
| 43 | $CH_3$ | 5,6-di—$CH_3$ | | H | H | H | $CH_2$—CHOH—$CH_2$ | H | $C_2H_5$ | $C_2H_5$ | Base | Öl |
| 44 | $CH_3$ | 4-Cl | H | H | H | H | $CH_2$—CHOH—$CH_2$ | H | $C_2H_5$ | $C_2H_5$ | HCl | 141–143 |
| 45 | $CH_3$ | 5-Cl | H | H | H | H | $CH_2$—CHOH—$CH_2$ | H | $C_2H_5$ | $C_2H_5$ | Cit. | 113–115 |
| 46 | $CH_3$ | 6-Cl | H | H | H | H | $CH_2$—CHOH—$CH_2$ | H | $C_2H_5$ | $C_2H_5$ | Cit. | 125–126 |

Fortsetzung

| Bsp. Nr. | $R_1$ | $R_3$ | $R_4$ | $R_5$ | $R_6$ | $R_7$ | Z | $R_2$ | $R_8$ | $R_9$ | Salz | Fp °C |
|---|---|---|---|---|---|---|---|---|---|---|---|---|
| 47 | H | H | H | H | H | H | $CH_2—CHOH—CH_2$ | H | $C_2H_5$ | $C_2H_5$ | HCl | 166–168 |
| 48 | 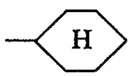 | H | H | H | H | H | $CH_2—CHOH—CH_2$ | H | $C_2H_5$ | $C_2H_5$ | | |
| 49 | $CH_2$— 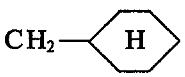 | H | H | H | H | H | $CH_2—CHOH—CH_2$ | H | $C_2H_5$ | $C_2H_5$ | | |
| 50 | $CH_2—CH{=}CH_2$ | H | H | H | H | H | $CH_2—CHOH—CH_2$ | H | $C_2H_5$ | $C_2H_5$ | | |
| 51 | $CH_2$— 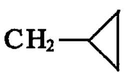 | H | H | H | H | H | $CH_2—CHOH—CH_2$ | H | $C_2H_5$ | $C_2H_5$ | | |

HCl = Hydrochlorid, HBr = Hydrobromid, Cit = Citrat, Base = freie Base.

## Beispiel I

### Tabletten

Man stellt Tabletten in folgender Zusammensetzung pro Tablette her:

| | |
|---|---|
| 2-[3-(N,N-Diäthylamino)-2-hydroxypropylaminocarbonyl]-3-methoxy-1-phenylindolhydrochlorid | 25 mg |
| Maisstärke | 60 mg |
| Milchzucker | 130 mg |
| Gelatine (als 10%ige Lösung) | 6 mg |

Der Wirkstoff, die Maisstärke und der Milchzucker werden mit der 10%igen Gelatine-Lösung eingedickt. Die Paste wird zerkleinert und das entstandene Granulat wird auf geeignetes Blech gebracht und bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Beispiel II

### Suppositorien

Man stellt Suppositorien mit folgender Zusammensetzung pro Zäpfchen her:

| | |
|---|---|
| 2-[3-(N,N-Diäthylamino)-2-hydroxypropylaminocarbonyl]-3-methoxy-1-phenylindolhydrochlorid | 25 mg |
| Kakaobutter | 1975 mg |

Der Wirkstoff und die feingeriebene Suppositorienmasse werden gründlich gemischt und das Gemisch dann geschmolzen. Aus der durch Rühren homogen gehaltenen Schmelze werden Suppositorien von 2 g gegossen.

## Beispiel III

### Injektionslösung

Es wird eine Injektionslösung mit folgender Zusammensetzung pro ml hergestellt:

| | |
|---|---|
| 2-[3-(N,N-Diäthylamino)-2-hydroxypropylaminocarbonyl]-3-methoxy-1-phenylindolhydrochlorid | 25 mg |
| Dimethylacetamid | 100 mg |
| Propylenglycol | 500 mg |
| Benzylalkohol | 15 mg |
| Äthanol | 100 mg |
| Wasser für Injektionszwecke ad 1 ml. | |

Der Wirkstoff wird in Dimethylacetamid gelöst und mit Benzylalkohol, Propylenglycol, Äthanol und Wasser versetzt. Man filtriert durch einen Kerzenfilter, füllt in geeignete Ampullen ab, verschließt und sterilisiert die Ampullen.

**Patentansprüche für die Vertragsstaaten BE, CH, DE, FR, GB, IT, LI, LU, NL, SE**

1. 1-Phenyl-2-aminocarbonylindol-Verbindungen der allgemeinen Formel I

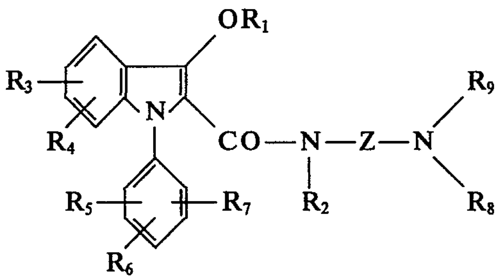

(I)

worin

$R_1$ Wasserstoff, eine Alkyl-, Alkenyl-, Cycloalkyl- oder Cycloalkylalkylgruppe mit bis zu 7 Kohlenstoffatomen bedeutet,
$R_2$ Wasserstoff oder Alkyl mit 1–4 Kohlenstoffatomen bedeutet,
$R_3$ Wasserstoff, Halogen, Alkyl mit 1–4 Kohlenstoffatomen, Hydroxy oder Alkoxy mit 1–4 Kohlenstoffatomen bedeutet,
$R_4$ Wasserstoff, Halogen, Alkyl mit 1–4 Kohlenstoffatomen, Hydroxy, Alkoxy mit 1–4 Kohlenstoffatomen oder, falls $R_3$ Wasserstoff ist, auch Nitro oder Trifluormethyl bedeutet, oder
$R_3$ und $R_4$ an benachbarte Kohlenstoffatome gebunden sind und zusammen Methylendioxy oder Äthylendioxy bedeuten,
$R_5$ Wasserstoff, Alkyl mit 1–4 Kohlenstoffatomen, Halogen, Hydroxy oder Alkoxy mit 1–4 Kohlenstoffatomen bedeutet,
$R_6$ Wasserstoff, Alkyl mit 1–4 Kohlenstoffatomen, Alkoxy mit 1–4 Kohlenstoffatomen, Halogen, Hydroxy oder, falls $R_5$ Wasserstoff ist, auch Nitro oder Trifluormethyl bedeutet, oder
$R_5$ und $R_6$ an benachbarte Kohlenstoffatome gebunden sind und zusammen Methylendioxy oder Äthylendioxy bedeuten,
$R_7$ Wasserstoff, oder, falls $R_5$ und $R_6$ Alkoxy mit 1–4 Kohlenstoffatomen sind, auch Alkoxy mit 1–4 Kohlenstoffatomen bedeutet,
$R_8$ Wasserstoff oder Alkyl mit 1–4 Kohlenstoffatomen bedeutet,
$R_9$ Wasserstoff oder Alkyl mit 1–4 Kohlenstoffatomen bedeutet, oder
$R_8$ und $R_9$ zusammen mit dem Stickstoffatom, an das sie gebunden sind, eine heterocyclische Gruppe a bilden

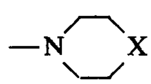

(a)

worin X für eine Bindung, $-CH_2-$, $-C_2H_4-$, O oder S steht,
Z eine Alkylenkette mit 2–5 Kohlenstoffatomen bedeutet, welche gegebenenfalls an einem nicht an Stickstoff gebundenen Kohlenstoff durch Hydroxy substituiert ist,

sowie deren Säureadditionssalze.

2. 1-Phenyl-2-aminocarbonylindol-Verbindungen gemäß Anspruch 1, worin Z und $R_1$ bis $R_9$ obige Bedeutung besitzen und $R_1$ Alkyl mit 1–4 Kohlenstoffatomen oder Cycloalkylalkyl und $R_2$ Wasserstoff bedeuten.

3. 1-Phenyl-2-aminocarbonylindol-Verbindungen gemäß Anspruch 2, worin Z und $R_1$ bis $R_7$ obige Bedeutung besitzen, $R_8$ Wasserstoffatom oder Alkyl mit 1–4 Kohlenstoffatomen bedeutet und $R_9$ Wasserstoff oder Alkyl mit 1–4 Kohlenstoffatomen bedeutet.

4. 1-Phenyl-2-aminocarbonylindol-Verbindungen gemäß Anspruch 1, worin Z obige Bedeutung besitzt, $R_1$ Wasserstoff oder Alkyl mit 1–4 Kohlenstoffatomen bedeutet, $R_2$ Wasserstoff bedeutet, $R_3$ Wasserstoff oder Halogen bedeutet, $R_4$ Wasserstoff oder Halogen bedeutet, $R_5$ Wasserstoff, Halogen oder Alkyl mit 1–4 Kohlenstoffatomen bedeutet, $R_6$ Wasserstoff, Halogen oder Alkyl mit 1–4 Kohlenstoffatomen bedeutet, $R_7$ Wasserstoff bedeutet, $R_8$ Wasserstoff oder Alkyl mit 1–4 Kohlenstoffatomen bedeutet und $R_9$ Wasserstoff oder Alkyl mit 1–4 Kohlenstoffatomen bedeutet.

5. 1-Phenyl-2-aminocarbonylindol-Verbindungen gemäß Anspruch 4, worin $R_1$ bis $R_9$ obige Bedeutung besitzen und Z eine Alkylenkette mit 2–5 Kohlenstoffatomen bedeutet, welche an einem nicht an Stickstoff gebundenen Kohlenstoff durch Hydroxy substituiert ist.

6. 5-Brom-2-[3-(N,N-diäthylamino)-2-hydroxypropylaminocarbonyl]-3-methoxy-1-phenylindol und dessen Säureadditionssalze gemäß Anspruch 5.

7. 2-[3-(N,N-Diäthylamino)-2-hydroxypropylaminocarbonyl]-3-hydroxy-1-phenylindol und dessen Säureadditionssalze gemäß Anspruch 5.

8. 2-[3-(N,N-Diäthylamino)-2-hydroxypropylaminocarbonyl]-3-methoxy-1-phenylindol und dessen Säureadditionssalze gemäß Anspruch 5.

9. Verbindungen der allgemeinen Formel IId

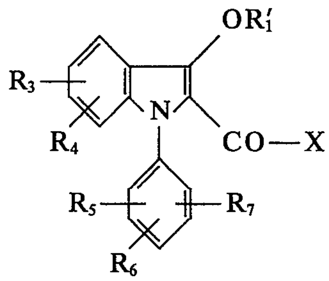

(IId)

worin $R_1'$ eine Alkyl-, Alkenyl, Cycloalkyl- oder Cycloalkylalkylgruppe mit bis zu 7 Kohlenstoffatomen bedeutet, $R_3$, $R_4$, $R_5$, $R_6$ und $R_7$ die in Anspruch 1 angegebene Bedeutung besitzen, und X Hydroxy oder eine reaktive Gruppe bedeutet.

10. Verbindungen gemäß Anspruch 9, worin $R_1'$, $R_3$, $R_4$, $R_5$, $R_6$ und $R_7$ obige Bedeutung besitzen und X für Hydroxy, Halogen, Alkoxy mit 1—4 Kohlenstoffatomen oder eine Gruppe O—CO—W steht, worin W niederes Alkyl oder niederes Alkoxy bedeutet.

11. Verfahren zur Herstellung von 1-Phenyl-2-aminocarbonylindol-Verbindungen der allgemeinen Formel I

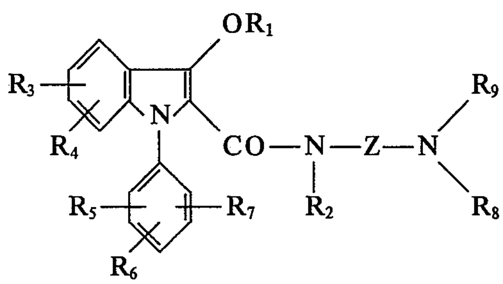

(I)

worin $R_1$, $R_2$, $R_3$, $R_4$, $R_5$, $R_6$, $R_7$, $R_8$, $R_9$ und Z die in Anspruch 1 angegebene Bedeutung besitzen, sowie deren Säureadditionssalzen, dadurch gekennzeichnet, daß man

a) Verbindungen der Formel II

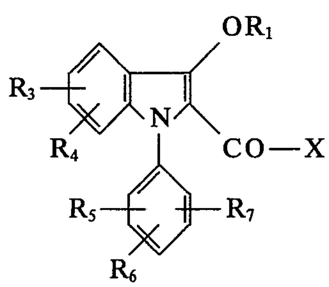

(II)

worin $R_1$, $R_3$, $R_4$, $R_5$, $R_6$ und $R_7$ obige Bedeutung besitzen und X Hydroxy oder eine reaktive Gruppe bedeutet,

mit einer Verbindung der Formel VI

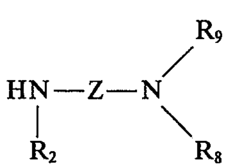

(VI)

worin $R_2$, $R_8$, $R_9$ und Z obige Bedeutung besitzen, umsetzt, oder

b) Verbindungen der Formel III

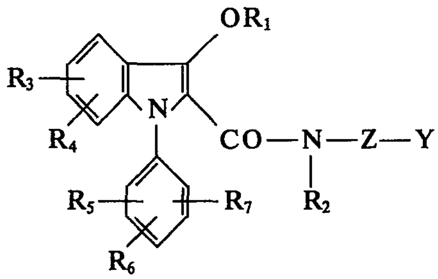

(III)

worin $R_1$, $R_2$, $R_3$, $R_4$, $R_5$, $R_6$, $R_7$ und Z obige Bedeutung besitzen und Y eine aminolytisch abspaltbare Gruppe bedeutet,

mit Verbindungen der Formel VII

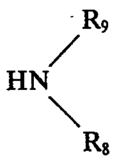

(VII)

worin $R_8$ und $R_9$ obige Bedeutung besitzen, umsetzt, oder

c) Verbindungen der Formel IVa

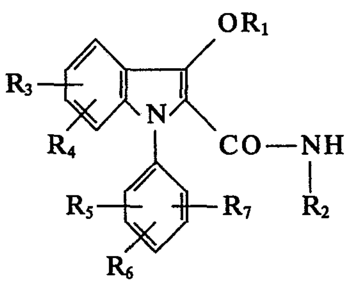

(IVa)

worin $R_1$, $R_2$, $R_3$, $R_4$, $R_5$, $R_6$ und $R_7$ obige Bedeutung besitzen,

mit Verbindungen der Formel VIII

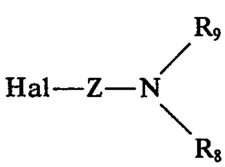

(VIII)

worin Z, $R_8$ und $R_9$ obige Bedeutung besitzen, und Hal Halogen bedeutet,

umsetzt, oder

d) zur Herstellung von Verbindungen der Formel Ia

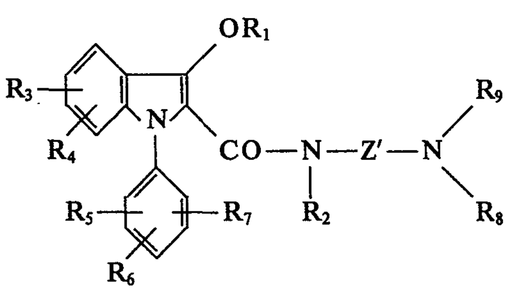

(Ia)

worin $R_1$, $R_2$, $R_3$, $R_4$, $R_5$, $R_6$, $R_7$, $R_8$ und $R_9$ obige Bedeutung besitzen, und Z' eine Alkylengruppe mit

2—5 Kohlenstoffatomen bedeutet, welche an einem nicht an Stickstoff gebundenen Kohlenstoffatom durch Hydroxy substituiert ist,

Verbindungen der Formel IV

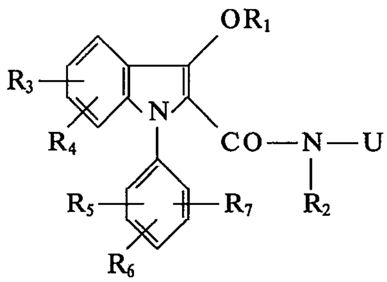

(IV)

worin $R_1$, $R_2$, $R_3$, $R_4$, $R_5$, $R_6$ und $R_7$ obige Bedeutung besitzen und U für Wasserstoff oder eine Gruppe der Formel V

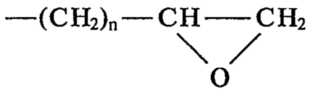

(V)

worin n 1—3 bedeutet, steht,

umsetzt mit einer Verbindung der Formel IX

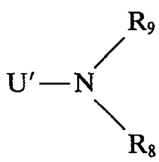

(IX)

worin $R_8$ und $R_9$ obige Bedeutung besitzen, und U', falls U Wasserstoff darstellt, für eine Gruppe der Formel V steht, oder, falls U eine Gruppe der Formel V darstellt, für Wasserstoff steht,

und gegebenenfalls freie Verbindungen der Formel I in ihre Säureadditionssalze überführt oder die Säureadditionssalze in die freien Verbindungen der Formel I überführt.

12. Arzneimittel enthaltend eine pharmakologisch wirksame Menge einer 1-Phenyl-2-aminocarbonylindol-Verbindung gemäß Anspruch 1 und übliche Hilfs- und/oder Trägerstoffe.

13. Verfahren zur Herstellung von Verbindungen der allgemeinen Formel IId

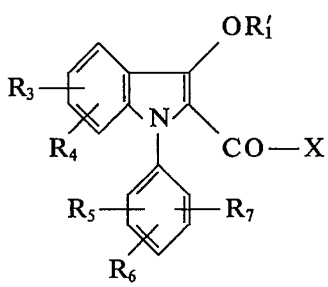

(IId)

worin $R_1'$, $R_3$, $R_4$, $R_5$, $R_6$, $R_7$ und X die in Anspruch 9 angegebene Bedeutung besitzen, dadurch gekennzeichnet, daß man

a') Verbindungen der Formel X

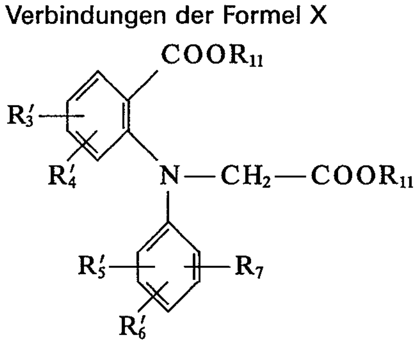

(X)

worin $R_7$ obige Bedeutung besitzt, und $R_{11}$ Alkyl mit 1—4 Kohlenstoffatomen bedeutet, $R_3'$, $R_4'$, $R_5'$ und $R_6'$ jeweils die für $R_3$, $R_4$, $R_5$ und $R_6$ angegebene Bedeutung besitzen, wobei jedoch eine freie Hydroxygruppe mit einer Schutzgruppe versehen ist, cyclisiert zu Verbindungen der Formel IIb

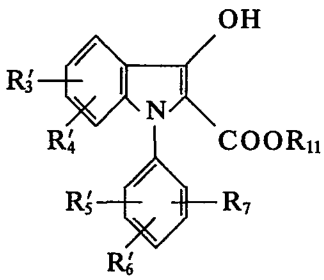

(IIb)

worin $R_3'$, $R_4'$, $R_5'$, $R_6'$, $R_7$ und $R_{11}$ obige Bedeutung besitzen, und diese zu Verbindungen der Formel IIc

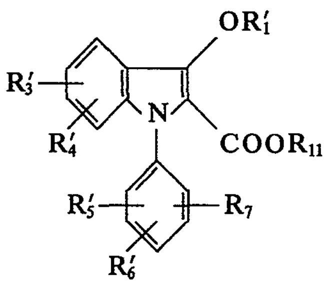

(IIc)

worin $R_1'$, $R_3'$, $R_4'$, $R_5'$, $R_6'$, $R_7$ und $R_{11}$ obige Bedeutung besitzen, veräthert, oder

b') zur Herstellung von Verbindungen der Formel IIc

Verbindungen der Formel XIa

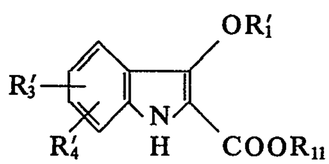

(XIa)

worin $R_1'$, $R_3'$, $R_4'$ und $R_{11}$ obige Bedeutung besitzen,

mit Verbindungen der Formel XII

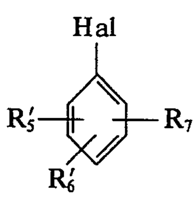

(XII)

worin $R_5'$, $R_6'$, $R_7$ und Hal obige Bedeutung besitzen, umsetzt und allfällige Hydroxyschutzgruppen abspaltet, und gewünschtenfalls die erhaltenen Ester der Formel IId, worin X die $OR_{11}$ Gruppe bedeutet, zu den entsprechenden Säuren der Formel IId hydrolysiert und diese gewünschtenfalls in ihre Säurederivate überführt.

**Patentansprüche für den Vertragsstaat AT**

1. Verfahren zur Herstellung von 1-Phenyl-2-aminocarbonylindol-Verbindungen der allgemeinen Formel I

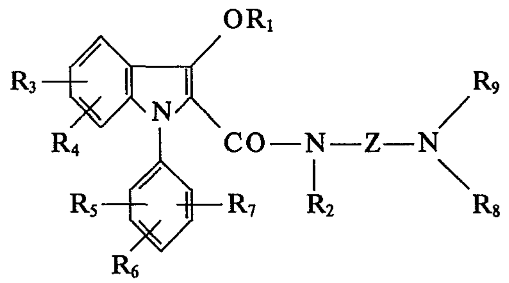

(I)

worin

$R_1$ Wasserstoff, eine Alkyl-, Alkenyl-, Cycloalkyl- oder Cycloalkylalkylgruppe mit bis zu 7 Kohlenstoffatomen bedeutet,
$R_2$ Wasserstoff oder Alkyl mit 1—4 Kohlenstoffatomen bedeutet,
$R_3$ Wasserstoff, Halogen, Alkyl mit 1—4 Kohlenstoffatomen, Hydroxy oder Alkoxy mit 1—4 Kohlenstoffatomen bedeutet,
$R_4$ Wasserstoff, Halogen, Alkyl mit 1—4 Kohlenstoffatomen, Hydroxy, Alkoxy mit 1—4 Kohlenstoffatomen oder, falls $R_3$ Wasserstoff ist, auch Nitro oder Trifluormethyl bedeutet, oder
$R_3$ und $R_4$ an benachbarte Kohlenstoffatome gebunden sind und zusammen Methylendioxy oder Äthylendioxy bedeuten,
$R_5$ Wasserstoff, Alkyl mit 1—4 Kohlenstoffatomen, Halogen, Hydroxy oder Alkoxy mit 1—4 Kohlenstoffatomen bedeutet,
$R_6$ Wasserstoff, Alkyl mit 1—4 Kohlenstoffatomen, Alkoxy mit 1—4 Kohlenstoffatomen, Halogen, Hydroxy oder, falls $R_5$ Wasserstoff ist, auch Nitro oder Trifluormethyl bedeutet, oder
$R_5$ und $R_6$ an benachbarte Kohlenstoffatome gebunden sind und zusammen Methylendioxy oder Äthylendioxy bedeuten,
$R_7$ Wasserstoff, oder, falls $R_5$ und $R_6$ Alkoxy mit 1—4 Kohlenstoffatomen sind, auch Alkoxy mit 1—4 Kohlenstoffatomen bedeutet,
$R_8$ Wasserstoff oder Alkyl mit 1—4 Kohlenstoffatomen bedeutet,
$R_9$ Wasserstoff oder Alkyl mit 1—4 Kohlenstoffatomen bedeutet, oder
$R_8$ und $R_9$ zusammen mit dem Stickstoffatom, an das sie gebunden sind, eine heterocyclische Gruppe a bilden,

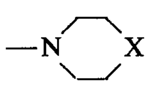

(a)

worin X für eine Bindung, $-CH_2-$, $-C_2H_4-$, O oder S steht,
Z eine Alkylenkette mit 2—5 Kohlenstoffatomen bedeutet, welche gegebenenfalls an einem nicht an Stickstoff gebundenen Kohlenstoff durch Hydroxy substituiert ist,

sowie deren Säureadditionssalze, dadurch gekennzeichnet, daß man

a) Verbindungen der Formel II

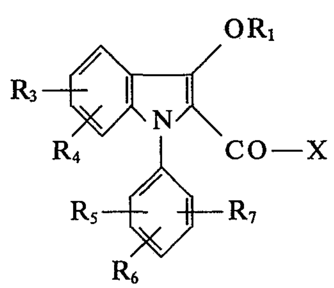

(II)

worin $R_1$, $R_3$, $R_4$, $R_5$, $R_6$ und $R_7$ obige Bedeutung besitzen und X Hydroxy oder eine reaktive Gruppe bedeutet,

mit einer Verbindung der Formel VI

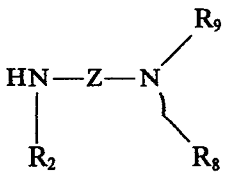

(VI)

worin $R_2$, $R_8$, $R_9$ und Z obige Bedeutung besitzen, umsetzt, oder

b) Verbindungen der Formel III

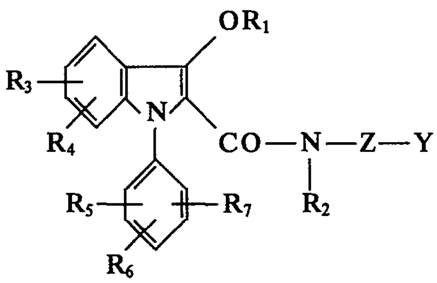

(III)

worin $R_1$, $R_2$, $R_3$, $R_4$, $R_5$, $R_6$, $R_7$ und Z obige Bedeutung besitzen und Y eine aminolytisch abspaltbare Gruppe bedeutet,

mit Verbindungen der Formel VII

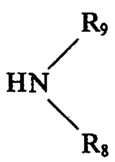

(VII)

worin $R_8$ und $R_9$ obige Bedeutung besitzen, umsetzt, oder

c) Verbindungen der Formel IVa

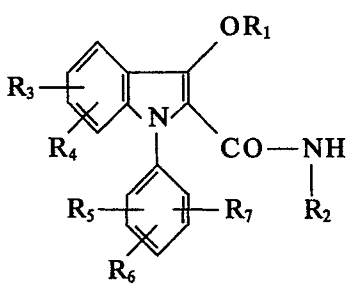

(IVa)

worin $R_1$, $R_2$, $R_3$, $R_4$, $R_5$, $R_6$ und $R_7$ obige Bedeutung besitzen,

mit Verbindungen der Formel VIII

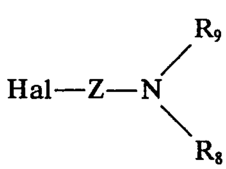

(VIII)

worin Z, $R_8$ und $R_9$ obige Bedeutung besitzen, und Hal Halogen bedeutet,

umsetzt, oder

d) zur Herstellung von Verbindungen der Formel Ia

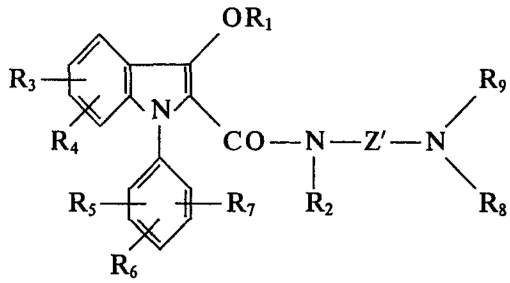

(Ia)

worin $R_1$, $R_2$, $R_3$, $R_4$, $R_5$, $R_6$, $R_7$, $R_8$ und $R_9$ obige Bedeutung besitzen, und Z' eine Alkylengruppe mit 2—5 Kohlenstoffatomen bedeutet, welche an einem nicht an Stickstoff gebundenen Kohlenstoffatom durch Hydroxy substituiert ist,

Verbindungen der Formel IV

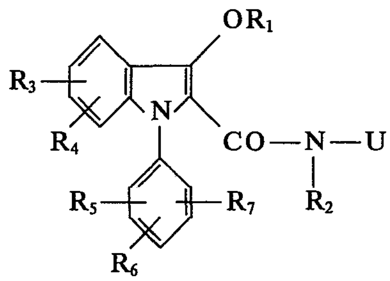

(IV)

worin $R_1$, $R_2$, $R_3$, $R_4$, $R_5$, $R_6$ und $R_7$ obige Bedeutung besitzen und U für Wasserstoff oder eine Gruppe der Formel V

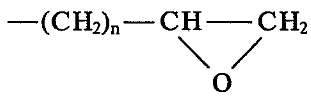

(V)

worin n 1—3 bedeutet, steht,

umsetzt mit einer Verbindung der Formel IX

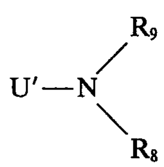

(IX)

worin $R_8$ und $R_9$ obige Bedeutung besitzen, und U', falls U Wasserstoff darstellt, für eine Gruppe der Formel V steht, oder, falls U eine Gruppe der Formel V darstellt, für Wasserstoff steht,

und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, daß man als Verbindungen der Formel I 1-Phenyl-2-aminocarbonylindol-Verbindungen herstellt, worin Z und $R_3$ bis $R_9$ obige Bedeutung besitzen und $R_1$ Alkyl mit 1—4 Kohlenstoffatomen oder Cycloalkylakyl und $R_2$ Wasserstoff bedeuten.

3. Verfahren nach Anspruch 2, dadurch gekennzeichnet, daß man als Verbindungen der Formel I 1-Phenyl-2-aminocarbonylindol-Verbindungen herstellt, worin Z und $R_1$ bis $R_7$ obige Bedeutung besitzen, $R_8$ Wasserstoff oder Alkyl mit 1—4 Kohlenstoffatomen bedeutet und $R_9$ Wasserstoff oder Alkyl mit 1—4 Kohlenstoffatomen bedeutet.

4. Verfahren nach Anspruch 1, dadurch gekennzeichnet, daß man als Verbindungen der Formel I 1-Phenyl-2-aminocarbonylindol-Verbindungen herstellt, worin Z obige Bedeutung besitzt, $R_1$ Wasserstoff oder Alkyl mit 1—4 Kohlenstoffatomen bedeutet, $R_2$ Wasserstoff bedeutet, $R_3$ Wasserstoff oder Halogen bedeutet, $R_4$ Wasserstoff oder Halogen bedeutet, $R_5$ Wasserstoff, Halogen oder Alkyl mit 1—4 Kohlenstoffatomen bedeutet, $R_6$ Wasserstoff, Halogen oder Alkyl mit 1—4 Kohlenstoffatomen bedeutet, $R_7$ Wasserstoff bedeutet, $R_8$ Wasserstoff oder Alkyl mit 1—4 Kohlenstoffatomen bedeutet und $R_9$ Wasserstoff oder Alkyl mit 1—4 Kohlenstoffatomen bedeutet.

5. Verfahren gemäß Anspruch 4, dadurch gekennzeichnet, daß man als Verbindungen der Formel I

1-Phenyl-2-aminocarbonylindol-Verbindungen herstellt, $R_1$ bis $R_7$ obige Bedeutung besitzen und Z eine Alkylenkette mit 2—5 Kohlenstoffatomen bedeutet, welche an einem nicht an Stickstoff gebundenen Kohlenstoff durch Hydroxy substituiert ist.

6. Verfahren nach Anspruch 5, dadurch gekennzeichnet, daß man als Verbindungen der Formel I 5-Brom-2-[3-(N,N-diäthylamino)-2-hydroxypropylaminocarbonyl]-3-methoxy-1-phenylindol und dessen Säureadditionssalze herstellt.

7. Verfahren nach Anspruch 5, dadurch gekennzeichnet, daß man als Verbindungen der Formel I 2-[3-(N,N-Diäthylamino)-2-hydroxypropylaminocarbonyl]-3-hydroxy-1-phenylindol und dessen Säureadditionssalze herstellt.

8. Verfahren nach Anspruch 5, dadurch gekennzeichnet, daß man als Verbindungen der Formel I 2-[3-(N,N-Diäthylamino)-2-hydroxypropylaminocarbonyl]-3-methoxy-1-phenylindol und dessen Säureadditionssalze herstellt.

9. Verfahren zur Herstellung von Verbindungen der allgemeinen Formel IId

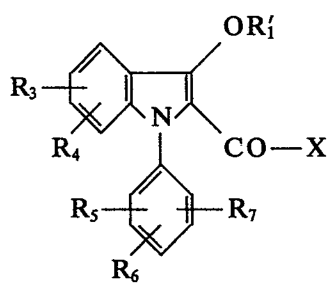

(IId)

worin
$R_1'$ eine Alkyl-, Alkenyl, Cycloalkyl- oder Cycloalkylalkylgruppe mit bis zu 7 Kohlenstoffatomen bedeutet und $R_3$, $R_4$, $R_5$, $R_6$, $R_7$ und X die in Anspruch 1 angegebene Bedeutung besitzen, dadurch gekennzeichnet, daß man

a') Verbindungen der Formel X

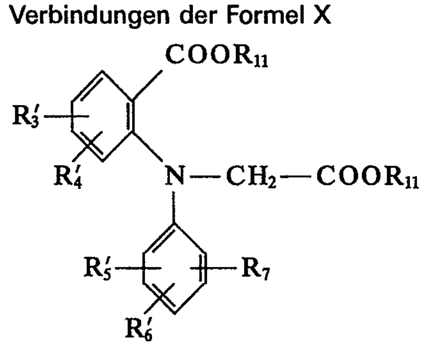

(X)

worin $R_7$ obige Bedeutung besitzt, und $R_{11}$ Alkyl mit 1—4 Kohlenstoffatomen bedeutet, $R_3'$, $R_4'$, $R_5'$ und $R_6'$ jeweils die für $R_3$, $R_4$, $R_5$ und $R_6$ angegebene Bedeutung besitzen, wobei jedoch eine freie Hydroxygruppe mit einer Schutzgruppe versehen ist, cyclisiert zu Verbindungen der Formel IIb

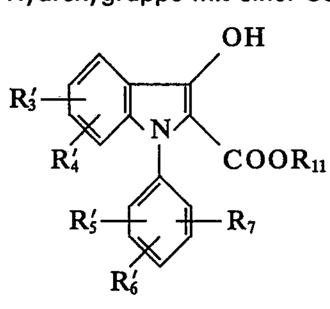

(IIb)

worin $R'_3$, $R'_4$, $R'_5$, $R'_6$, $R_7$ und $R_{11}$ obige Bedeutung besitzen, und diese zu Verbindungen der Formel IIc

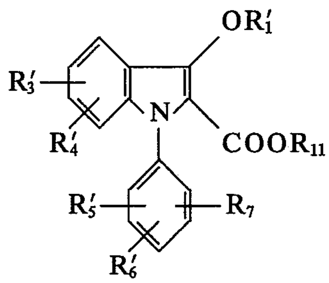

(IIc)

worin $R'_1$, $R'_3$, $R'_4$, $R'_5$, $R'_6$, $R_7$ und $R_{11}$ obige Bedeutung besitzen, veräthert, oder

b') zur Herstellung von Verbindungen der Formel IIc

Verbindungen der Formel XIa

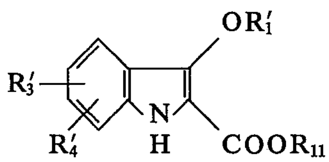

(XIa)

worin $R'_1$, $R'_3$, $R'_4$ und $R_{11}$ obige Bedeutung besitzen,

mit Verbindungen der Formel XII

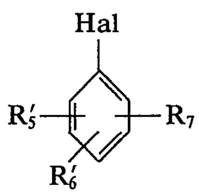

(XII)

worin $R'_5$, $R'_6$, $R'_7$ und Hal obige Bedeutung besitzen, umsetzt und allfällige Hydroxyschutzgruppen abspaltet, und gewünschtenfalls die erhaltenen Ester der Formel IId, worin X die $OR_{11}$-Gruppe bedeutet, zu den entsprechenden Säuren der Formel IId hydrolysiert und diese gewünschtenfalls in ihre Säurederivate überführt.

10. Verfahren nach Anspruch 9, dadurch gekennzeichnet, daß man Verbindungen der Formel IId herstellt, worin $R'_1$, $R_3$, $R_4$, $R_5$, $R_6$ und $R_7$ obige Bedeutung besitzen und X für Hydroxy, Halogen, Alkoxy mit 1—4 Kohlenstoffatomen oder eine Gruppe O—CO—W steht, worin W niederes Alkyl oder niederes Alkoxy bedeutet.

**Claims for the contracting states: BE, CH, DE, FR, GB, IT, LI, LU, NL, SE**

1. 1-Phenyl-2-aminocarbonylindole compounds of the general formula I

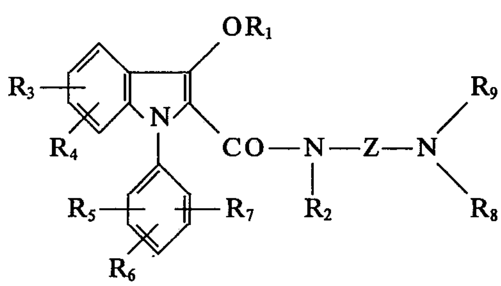

(I)

wherein

$R_1$ is hydrogen, an alkyl, alkenyl, cycloalkyl or cycloalkylalkyl group with up to 7 carbon atoms,
$R_2$ is hydrogen or alkyl with 1—4 carbon atoms,

$R_3$ is hydrogen, halogen, alkyl with 1—4 carbon atoms, hydroxy or alkoxy with 1—4 carbon atoms,

$R_4$ is hydrogen, halogen, alkyl with 1—4 carbon atoms, hydroxy, alkoxy with 1—4 carbon atoms, or, if $R_3$ is hydrogen, also nitro or trifluoromethyl, or

$R_3$ and $R_4$ are bonded to adjacent carbon atoms and together denote methylenedioxy or ethylenedioxy,

$R_5$ is hydrogen, alkyl with 1—4 carbon atoms, halogen, hydroxy or alkoxy with 1—4 carbon atoms,

$R_6$ is hydrogen, alkyl with 1—4 carbon atoms, alkoxy with 1—4 carbon atoms, halogen, hydroxy or, if $R_5$ is hydrogen, also nitro or trifluoromethyl, or

$R_5$ and $R_6$ are bonded to adjacent carbon atoms and together denote methylenedioxy or ethylenedioxy,

$R_7$ is hydrogen or, if $R_5$ and $R_6$ are alkoxy with 1—4 carbon atoms, also alkoxy with 1—4 carbon atoms,

$R_8$ is hydrogen or alkyl with 1—4 carbon atoms,

$R_9$ is hydrogen or alkyl with 1—4 carbon atoms, or

$R_8$ and $R_9$, together with the nitrogen atom to which they are bonded, form a heterocyclic group a

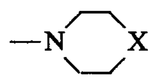

(a)

wherein X represents a bond, $-CH_2-$, $-C_2H_4-$, O or S,

Z is an alkylene chain with 2—5 carbon atoms, which is optionally substituted by hydroxyl on a carbon atom which is not bonded to nitrogen,

as well as their acid additions salts.

2. 1-Phenyl-2-aminocarbonylindole compounds according to Claim 1, wherein Z and $R_3$ to $R_9$ have the above meanings and $R_1$ is alkyl with 1—4 carbon atoms or cycloalkylalkyl, and $R_2$ is hydrogen.

3. 1-Phenyl-2-aminocarbonylindole compounds according to Claim 2, wherein Z and $R_1$ to $R_7$ have the above meanings, $R_8$ is hydrogen or alkyl with 1—4 carbon atoms and $R_9$ is hydrogen or alkyl with 1—4 carbon atoms.

4. 1-Phenyl-2-aminocarbonylindole compounds according to Claim 1, wherein Z has the above meaning, $R_1$ is hydrogen or alkyl with 1—4 carbon atoms, $R_2$ is hydrogen, $R_3$ is hydrogen or halogen, $R_4$ is hydrogen or halogen, $R_5$ is hydrogen, halogen or alkyl with 1—4 carbon atoms, $R_6$ is hydrogen, halogen or alkyl with 1—4 carbon atoms, $R_7$ is hydrogen, $R_8$ is hydrogen or alkyl with 1—4 carbon atoms and $R_9$ is hydrogen or alkyl with 1—4 carbon atoms.

5. 1-Phenyl-2-aminocarbonylindole compounds according to Claim 4, wherein $R_1$ to $R_9$ have the above meanings and Z is an alkylene chain with 2—5 carbon atoms and is substituted by hydroxy on a carbon atom which is not bonded to nitrogen.

6. 5-Bromo-2-(3-(N,N-diethylamino)-2-hydroxypropylaminocarbonyl)-3-methoxy-1-phenylindole and its acid addition salts according to Claim 5.

7. 2-(3-(N,N-diethylamino)-2-hydroxypropylaminocarbonyl)-3-hydroxy-1-phenylindole and its acid addition salts according to Claim 5.

8. 2-(3-(N,N-Diethylamino)-2-hydroxypropylaminocarbonyl)-3-methoxy-1-phenylindole and its acid addition salts according to Claim 5.

9. Compounds of the general formula IId

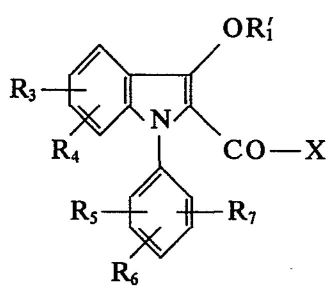

(IId)

wherein $R_1'$ is an alkyl, alkenyl, cycloalkyl or cycloalkylakyl group with up to 7 carbon atoms, $R_3$, $R_4$, $R_5$, $R_6$ and $R_7$ have the meanings given in Claim 1, and X is hydroxy or a reactive group.

10. Compounds according to Claim 9, wherein $R_1'$, $R_3$, $R_4$, $R_5$, and $R_6$ and $R_7$ have the above meanings and X is hydroxy, halogen, alkoxy with 1—4 carbon atoms, or an O—CO—W group, wherein W is lower alkyl or lower alkoxy.

11. Process for the preparation of 1-phenyl-2-aminocarbonylindole compounds of the general formula I

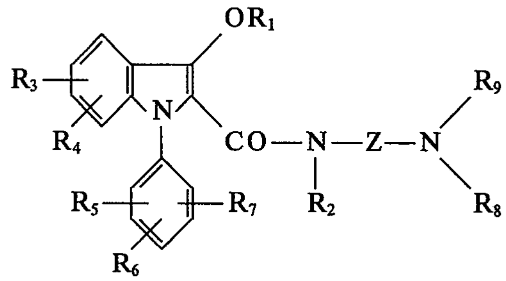

(I)

wherein $R_1$, $R_2$, $R_3$, $R_4$, $R_5$, $R_6$, $R_7$, $R_8$, $R_9$ and Z have the meanings given in Claim 1, as well as their acid addition salts, characterised in that,

a) compounds of the formula II

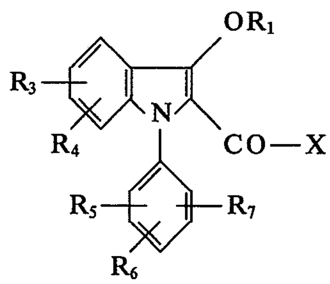

(II)

wherein $R_1$, $R_3$, $R_4$, $R_5$, $R_6$ and $R_7$ have the above meanings and X is hydroxy or a reactive group, are reacted

with a compound of the formula VI

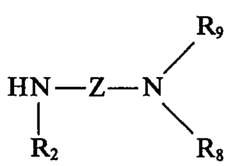

(VI)

wherein $R_2$, $R_8$, $R_9$ and Z have the above meanings, or

b) compounds of the formula III

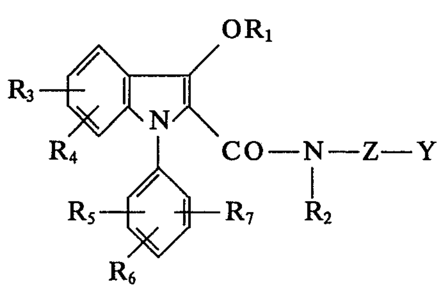

(III)

wherein $R_1$, $R_2$, $R_3$, $R_4$, $R_5$, $R_6$, $R_7$ and Z have the above meanings and Y denotes a group which can be split off aminolytically, are reacted

with compounds of the formula VII

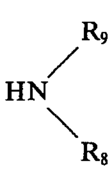

(VII)

wherein $R_8$ and $R_9$ have the above meanings, or

c) compounds of the formual IVa

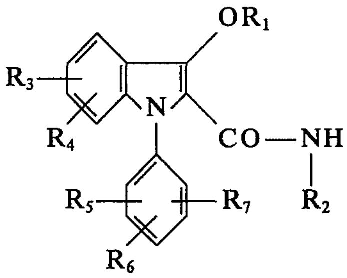

(IVa)

wherein $R_1$, $R_2$, $R_3$, $R_4$, $R_5$, $R_6$ and $R_7$ have the above meanings, are reacted with compounds of the formula VIII

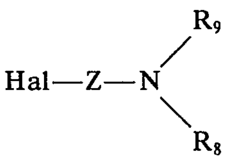

(VIII)

wherein Z, $R_8$ and $R_9$ have the above meanings and Hal is halogen, or

d) for the preparation of the compounds of the formula Ia

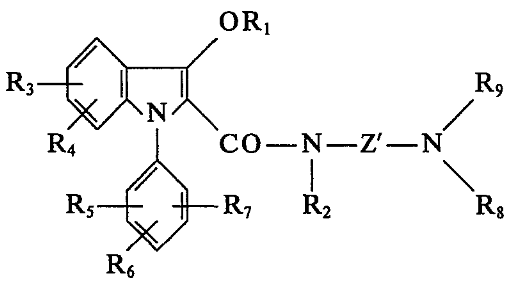

(Ia)

wherein $R_1$, $R_2$, $R_3$, $R_4$, $R_5$, $R_6$, $R_7$, $R_8$ and $R_9$ have the above meanings and Z' is an alkylene group with 2–5 carbon atoms and is substituted by hydroxy on a carbon atom which is not bonded to nitrogen,

compounds of the formula IV

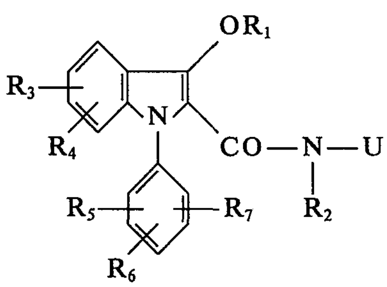

(IV)

wherein $R_1$, $R_2$, $R_3$, $R_4$, $R_5$, $R_6$ and $R_7$ have the above meanings and U is hydrogen or a group of the formula V

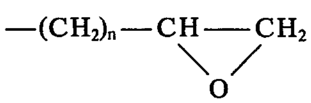

(V)

wherein n 1–3, are reacted

with a compound of the formula IX

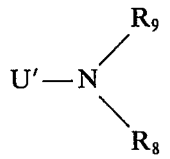

(IX)

wherein $R_8$ and $R_9$ have the above meanings and, if U is hydrogen, U' is a group of the formula V, or, if U is a group of the formula V, U' hydrogen,

and, if necessary, free compounds of the formula I are converted into their acid addition salts or the acid addition salts are converted into the free compounds of the formula I.

12. A pharmaceutical composition containing a pharmacologically active amount of a 1-phenyl-2-aminocarbonylindole compound according to Claim 1 and a conventional auxiliary or carrier material.

13. Process for the preparation of compounds of the general formula IId

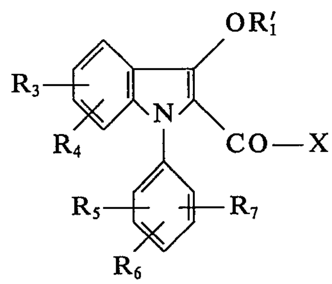

(IId)

wherein $R'_1$, $R_3$, $R_4$, $R_5$, $R_6$ and $R_7$ and X have the meanings given in Claim 9, characterised in that

a') compounds of the formula X

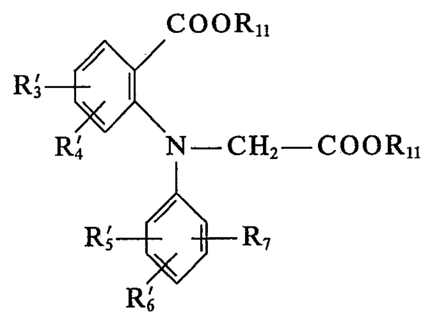

(X)

wherein $R_7$ has the above meaning, and $R_{11}$ is alkyl with 1—4 carbon atoms, each of $R'_3$, $R'_4$, $R'_5$, and $R'_6$ has the meaning defined for $R_3$, $R_4$, $R_5$ and $R_6$, but any free hydroxy group being provided with a protective group, are cyclised to compounds of the formula IIb

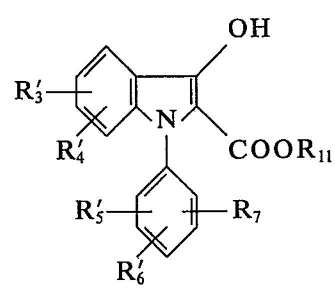

(IIb)

wherein $R'_3$, $R'_4$, $R'_5$, $R'_6$, $R_7$ and $R_{11}$ have the above meanings, and these are etherified to compounds of the formula IIc

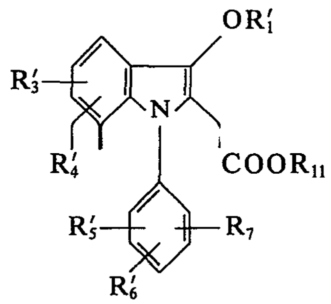

(IIc)

wherein $R_1'$, $R_3'$, $R_4'$, $R_5'$, $R_6'$, $R_7$ and $R_{11}$ have the above meanings, or

b') for the preparation of compounds of the formula IIc, compounds of the formula XIa

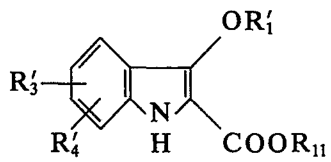

(XIa)

wherein $R_1'$, $R_3'$, $R_4'$ and $R_{11}$ have the above meanings, are reacted

with compounds of the formula XII

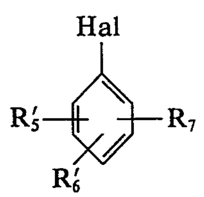

(XII)

wherein $R_5'$, $R_6'$, $R_7$ and Hal have the above meanings and any hydroxy-protective groups present are split off, and, if desired, the resulting ester of the formula IId wherein X is the $OR_{11}$ group is hydrolysed to the corresponding acid of the formula IId and, if desired, this is converted into its acid derivative.

**Claims for the contracting state: AT**

1. Process for the preparation of 1-Phenyl-2-aminocarbonylindole compounds of the general formula I

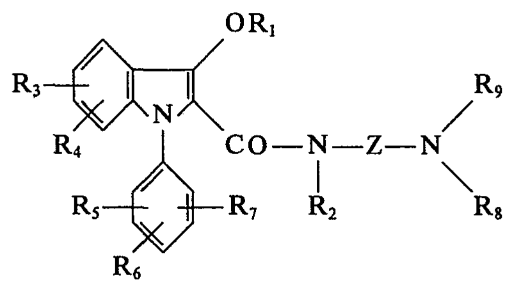

(I)

wherein

| | |
|---|---|
| $R_1$ | is hydrogen, an alkyl, alkenyl, cycloalkyl or cycloalkylalkyl group with up to 7 carbon atoms, |
| $R_2$ | is hydrogen or alkyl with 1–4 carbon atoms, |
| $R_3$ | is hydrogen, halogen, alkyl with 1–4 carbon atoms, hydroxy or alkoxy with 1–4 carbon atoms, |
| $R_4$ | is hydrogen, halogen, alkyl with 1–4 carbon atoms, hydroxy, alkoxy with 1–4 carbon atoms, or, if $R_3$ is hydrogen, also nitro or trifluoromethyl, or |
| $R_3$ and $R_4$ | are bonded to adjacent carbon atoms and together denote methylenedioxy or ethylenedioxy, |
| $R_5$ | is hydrogen, alkyl with 1–4 carbon atoms, halogen, hydroxy or alkoxy with 1–4 carbon atoms, |
| $R_6$ | is hydrogen, alkyl with 1–4 carbon atoms, alkoxy with 1–4 carbon atoms, halogen, hydroxy or, if $R_5$ is hydrogen, also nitro or trifluoromethyl, or |

$R_5$ and $R_6$ are bonded to adjacent carbon atoms and together denote methylenedioxy or ethylenedioxy,
$R_7$ is hydrogen or, if $R_5$ and $R_6$ are alkoxy with 1—4 carbon atoms, also alkoxy with 1—4 carbon atoms,
$R_8$ is hydrogen or alkyl with 1—4 carbon atoms,
$R_9$ is hydrogen or alkyl with 1—4 carbon atoms, or
$R_8$ and $R_9$, together with the nitrogen atom to which they are bonded, form a heterocyclic group a

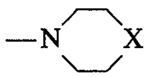

(a)

wherein X represents a bond, $-CH_2-$, $-C_2H_4-$, O or S,

Z is an alkylene chain with 2—5 carbon atoms, which is optionally substituted by hydroxyl on a carbon atom which is not bonded to nitrogen,

as well as their acid additions salts, characterised in that

a) compounds of the formula II

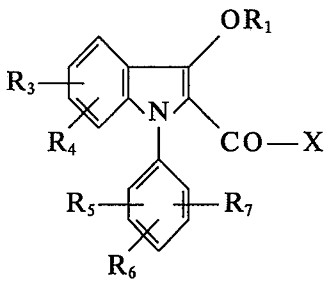

(II)

wherein $R_1$, $R_3$, $R_4$, $R_5$, $R_6$ and $R_7$ have the above meanings and X is hydroxy or a reactive group, are reacted

with a compound of the formula VI

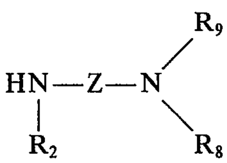

(VI)

wherein $R_2$, $R_8$, $R_9$ and Z have the above meanings, or

b) compounds of the formula III

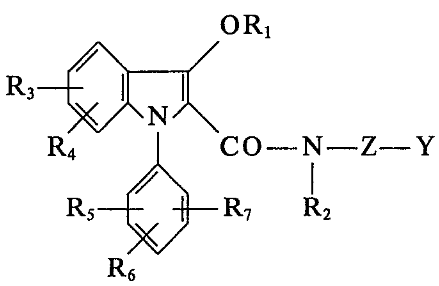

(III)

wherein $R_1$, $R_2$, $R_3$, $R_4$, $R_5$, $R_6$, $R_7$ and Z have the above meanings and Y denotes a group which can be split off aminolytically, are reacted

with compounds of the formula VII

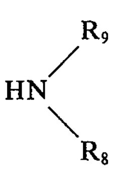

(VII)

wherein $R_8$ and $R_9$ have the above meanings, or

c) compounds of the formula IVa

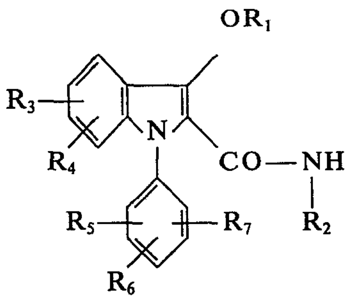

(IVa)

wherein $R_1$, $R_2$, $R_3$, $R_4$, $R_5$, $R_6$ and $R_7$ have the above meanings, are reacted

with compounds of the formula VIII

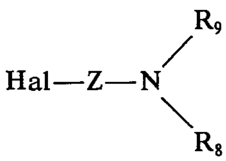

(VIII)

wherein Z, $R_8$ and $R_9$ have the above meanings and Hal is halogen, or

d) for the preparation of the compounds of the formula Ia

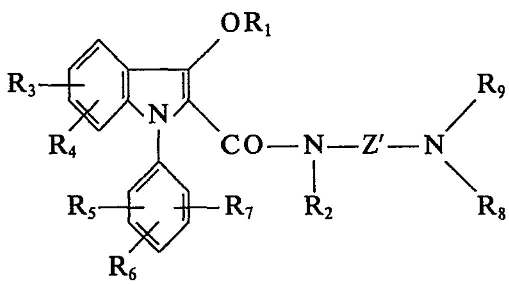

(Ia)

wherein $R_1$, $R_2$, $R_3$, $R_4$, $R_5$, $R_6$, $R_7$, $R_8$ and $R_9$ have the above meanings and Z′ is an alkylene group with 2–5 carbon atoms and is substituted by hydroxy on a carbon atom which is not bonded to nitrogen,

compounds of the formula IV

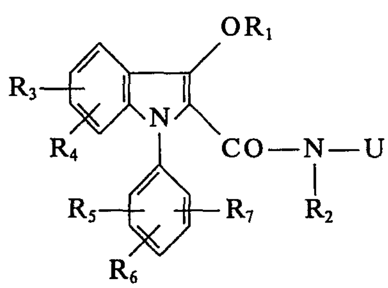

(IV)

wherein $R_1$, $R_2$, $R_3$, $R_4$, $R_5$, $R_6$ and $R_7$ have the above meanings and U is hydrogen or a group of the formula V

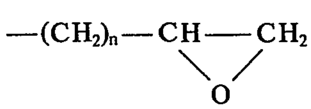

(V)

wherein n 1–3, are reacted

with a compound of the formula IX

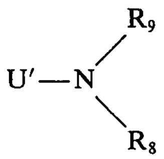

(IX)

wherein $R_8$ and $R_9$ have the above meanings and, if U is hydrogen, U' is a group of the formula V, or, if U is a group of the formula V, U' is hydrogen,

and, if necessary, free compounds of the formula I are converted into their acid addition salts or the acid addition salts are converted into the free compounds of the formula I.

2. Process according to Claim 1, characterized in that, as compounds of the formula I 1-Phenyl-2-aminocarbonylindole compounds are produced, wherein Z and $R_3$ to $R_9$ have the above meanings and $R_1$ is alkyl with 1—4 carbon atoms or cycloalkylalkyl and $R_2$ is hydrogen.

3. Process according to Claim 2, characterized in that, as compounds of the formula I 1-Phenyl-2-aminocarbonylindole compounds are produced, wherein Z and $R_1$ to $R_7$ have the above meanings, $R_8$ is hydrogen or alkyl with 1—4 carbon atoms and $R_9$ is hydrogen or alkyl with 1—4 carbon atoms.

4. Process according to Claim 1, characterized in that, as compounds of the formula I 1-Phenyl-2-aminocarbonylindole compounds are produced, wherein Z has the above meaning, $R_1$ is hydrogen or alkyl with 1—4 carbon atoms, $R_2$ is hydrogen, $R_3$ is hydrogen or halogen, $R_4$ is hydrogen or halogen, $R_5$ is hydrogen, halogen or alkyl with 1—4 carbon atoms, $R_6$ is hydrogen, halogen or alkyl with 1—4 carbon atoms, $R_7$ is hydrogen, $R_8$ is hydrogen or alkyl with 1—4 carbon atoms and $R_9$ is hydrogen or alkyl with 1—4 carbon atoms.

5. Prozess according to Claim 4, characterized in that, as compounds of the formula I 1-Phenyl-2-aminocarbonylindole compounds are produced, wherein $R_1$ to $R_9$ have the above meanings and Z is an alkylene chain with 2—5 carbon atoms and is substituted by hydroxy on a carbon atom which is not bonded to nitrogen.

6. Process according to Claim 5, characterized in that, as compounds of the formula I 5-bromo-2-(3-(N,N-diethylamino)-2-hydroxypropylaminocarbonyl)-3-methoxy-1-phenylindole and its acid addition salts are produced.

7. Process according to Claim 5, characterized in that, as compounds of the formula I 2-(3-(N,N-diethylamino)-2-hydroxypropylaminocarbonyl)-3-hydroxy-1-phenylindole and its acid addition salts are produced.

8. Process according to Claim 5, characterized in that, as compounds of the formula I 2-(3-(N,N-diethylamino)-2-hydroxypropylaminocarbonyl)-3-methoxy-1-phenylindole and its acid addition salts are produced.

9. Process for the preparation of compounds of the general formula IId

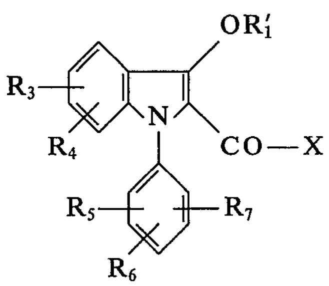

(IId)

wherein $R'_1$, is an alkyl, alkenyl, cycloalkyl or cycloalkylalkyl group with up to 7 carbon atoms and $R_3$, $R_4$, $R_5$, $R_6$, $R_7$ and X have the meanings given in Claim 1, characterized in that

a') compounds of the formula X

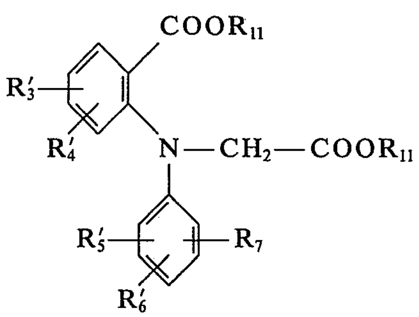

(X)

wherein $R_7$ has the above meaning, and $R_{11}$ is alkyl with 1—4 carbon atoms, each of $R'_3$, $R'_4$, $R'_5$ and $R'_6$ has the meaning defined for $R_3$, $R_4$, $R_5$ and $R_6$, but any free hydroxy group being provided with a protective group, are cyclised to compounds of the formula IIb

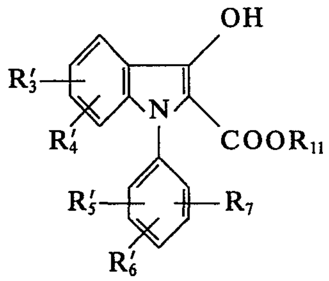

(IIb)

wherein $R'_3$, $R'_4$, $R'_5$, $R'_6$, $R_7$ and $R_{11}$ have the above meanings, and these are etherified to compounds of the formula IIc

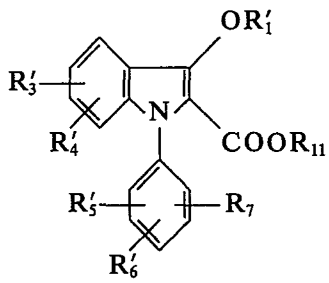

(IIc)

wherein $R'_1$, $R'_3$, $R'_4$, $R'_5$, $R'_6$, $R_7$ and $R_{11}$ have the above meanings, or

b') for the preparation of compounds of the formula IIc, compounds of the formula XIa

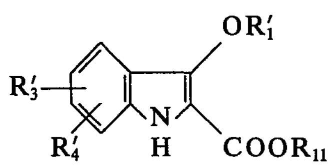

(XIa)

wherein $R'_1$, $R'_3$, $R'_4$, and $R_{11}$ have the above meanings, are reacted

with compounds of the formula XII

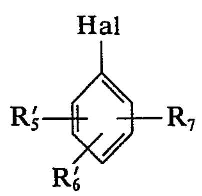

(XII)

wherein $R'_5$, $R'_6$, $R_7$ and Hal have the above meanings and any hydroxy-protective groups present are split off, and, if desired, the resulting ester of the formula IId wherein X is the $OR_{11}$ group is hydrolysed to the corresponding acid of the formula IId and, if desired, this is converted into its acid derivative.

10. Process according to Claim 9, characterized in that, compounds of the formula IId are produced, wherein $R'_1$, $R_3$, $R_4$, $R_5$, $R_6$, and $R_9$ have the above meanings and X is hydroxy, halogen, alkoxy with 1—4 carbon atoms or an O—CO—W group, wherein W is lower alkyl or lower alkoxy.

**Revendications pour les Etats contractants BE, CH, DE, FR, GB, IT, LI, LU, NL, SE**

1. Composés de 1-phényl-2-aminocarbonylindole de formule générale I

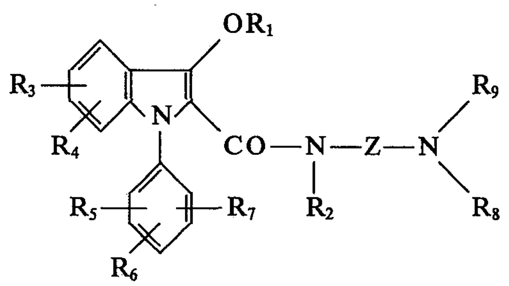

(I)

dans laquelle

$R_1$ représente l'hydrogène, un groupe alkyle, alcényle, cycloalkyle ou cycloalkylalkyle avec jusqu'à 7 atomes de carbone,

$R_2$ représente l'hydrogène, ou un alkyle avec 1 à 4 atomes de carbone,

$R_3$ représente l'hydrogène, un halogène, un alkyle avec 1 à 4 atomes de carbone, un hydroxy ou un alcoxy avec 1 à 4 atomes de carbone,

$R_4$ représente l'hydrogène, un halogène, un alkyle avec 1 à 4 atomes de carbone, un hydroxy, un alcoxy avec 1 à 4 atomes de carbone ou, au cas où $R_3$ est l'hydrogène, également un nitro ou un trifluorométhyle ou

$R_3$ et $R_4$ sont fixés sur des atomes de carbone voisins et représentent ensemble un méthylènedioxy ou un éthylènedioxy,

$R_5$ représente l'hydrogène, un alkyle avec 1 à 4 atomes de carbone, un halogène, un hydroxy ou un alcoxy avec 1 à 4 atomes de carbone,

$R_6$ représente l'hydrogène, un alkyle avec 1 à 4 atomes de carbone, un alcoxy avec 1 à 4 atomes de carbone, un halogène, un hydroxy ou, au cas où $R_5$ est l'hydrogène, également un nitro ou un trifluorométhyle ou

$R_5$ et $R_6$ sont fixés sur des atomes de carbone voisins et représentent ensemble un méthylènedioxy ou un éthylènedioxy,

$R_7$ représente l'hydrogène ou, au cas où $R_5$ et $R_6$ sont des alcoxy avec 1 à 4 atomes de carbone, également un alcoxy avec 1 à 4 atomes de carbone,

$R_8$ représente l'hydrogène ou un alkyle avec 1 à 4 atomes de carbone,

$R_9$ représente l'hydrogène ou un alkyle avec 1 à 4 atomes de carbone ou

$R_8$ et $R_9$ représentent ensemble, avec l'atome d'azote auquel ils sont liés, un groupe hétérocyclique a

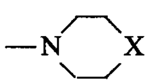

(a)

où X est une liaison, $-CH_2-$, $-C_2H_4-$, O ou S,

Z représente une chaîne alkylène avec 2 à 5 atomes de carbone qui est éventuellement substituée par un hydroxy sur un

carbone non lié à de l'azote, ainsi que leurs sels d'addition avec des acides.

2. Composés de 1-phényl-2-aminocarbonylindole selon la revendication 1, dans lesquels Z et $R_3$ à $R_9$ ont la signification précédente, $R_1$ représente un alkyle avec 1 à 4 atomes de carbone ou un cycloalkylalkyle et $R_2$ l'hydrogène.

3. Composés de 1-phényl-2-aminocarbonylindole selon la revendication 2, dans lesquels Z et $R_1$ à $R_7$ ont la signification précédente, $R_8$ représente l'hydrogène ou un alkyle avec 1 à 4 atomes de carbone et $R_9$ représente l'hydrogène ou un alkyle avec 1 à 4 atomes de carbone.

4. Composés de 1-phényl-2-aminocarbonylindole selon la revendication 1 dans lesquels Z a la signification précédente, $R_1$ représente l'hydrogène ou un alkyle avec 1 à 4 atomes de carbone, $R_2$ représente l'hydrogène, $R_3$ représente l'hydrogène ou un halogène, $R_4$ représente l'hydrogène ou un halogène, $R_5$ représente l'hydrogène, un halogène ou un alkyle avec 1 à 4 atomes de carbone, $R_6$ représente l'hydrogène, un halogène ou un alkyle avec 1 à 4 atomes de carbone, $R_7$ représente l'hydrogène, $R_8$ représente l'hydrogène ou un alkyle avec 1 à 4 atomes de carbone et $R_9$ représente l'hydrogène ou un alkyle avec 1 à 4 atomes de carbone.

5. Composés de 1-phényl-2-aminocarbonylindole selon la revendication 4, dans lesquels $R_1$ à $R_9$ ont la signification précédente et Z représente une chaîne alkylène avec 2 à 5 atomes de carbone qui est substituée par un hydroxy sur un carbone non lié à de l'azote.

6. 5-bromo-2-[3-(N,N-diéthylamino)-2-hydroxypropylaminocarbonyl]-3-méthoxy-1-phénylindole et ses sels d'addition avec des acides selon la revendication 5.

7. 2-[3-(N,N-diéthylamino)-2-hydroxypropylaminocarbonyl]-3-hydroxy-1-phénylindole et ses sels d'addition avec des acides selon la revendication 5.

8. 2-[3-(N,N-diéthylamino)-2-hydroxypropylaminocarbonyl]-3-méthoxy-1-phénylindole et ses sels d'addition avec des acides selon la revendication 5.

9. Composés de formule générale IId

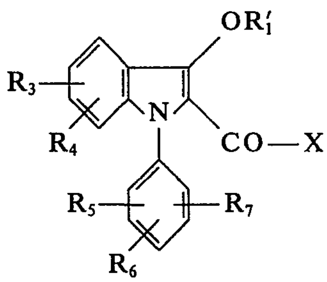

(IId)

dans laquelle $R'_1$ représente un groupe alkyle, alcényle, cycloalkyle ou cycloalkylalkyle avec jusqu'à 7 atomes de carbone, $R_3$, $R_4$, $R_5$, $R_6$ et $R_7$ ont la signification indiquée dans la revendication 1 et X représente un hydroxy ou un groupe réactif.

10. Composés selon la revendication 9, dans lesquels $R'_1$, $R_3$, $R_4$, $R_5$, $R_6$ et $R_7$ ont la signification précédente et X est un hydroxy, un halogène, un alcoxy avec 1 à 4 atomes de carbone ou un groupe O—CO—W où W représente un alkyle inférieur ou un alcoxy inférieur.

11. Procédé pour la préparation de composés de 1-phényl-2-aminocarbonylindole de formule générale I

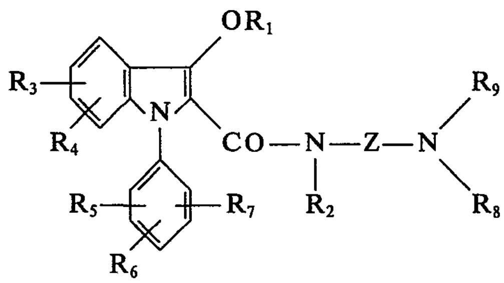

(I)

dans laquelle $R_1$, $R_2$, $R_3$, $R_4$, $R_5$, $R_6$, $R_7$, $R_8$, $R_9$ et Z ont la signification indiquée dans la revendication 1, ainsi que leurs sels d'addition avec des acides, caractérisé en ce que l'on fait réagir

a) des composés de formule II

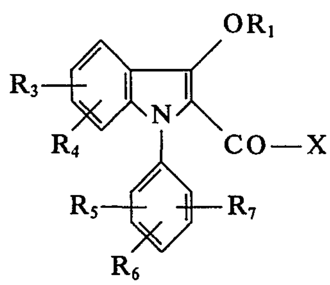

(II)

dans laquelle $R_1$, $R_3$, $R_4$, $R_5$, $R_6$ et $R_7$ ont la signification précédente et X représente un hydroxy ou un groupe réactif,

avec un composé de formule VI

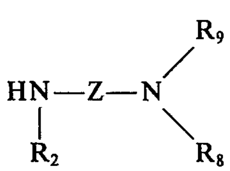

(VI)

dans laquelle $R_2$, $R_8$, $R_9$ et Z ont la signification précédente, ou que l'on fait réagir

b) des composés de formule III

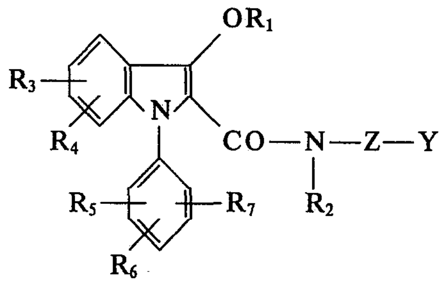

(III)

dans laquelle $R_1$, $R_2$, $R_3$, $R_4$, $R_5$, $R_6$, $R_7$ et Z ont la signification précédente et Y représente un groupe éliminable par aminolyse,

avec des composés de formule VII

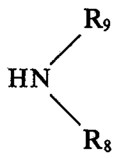

(VII)

dans laquelle $R_8$ et $R_9$ ont la signification précédente, ou que l'on fait réagir

c) des composés de formule IVa

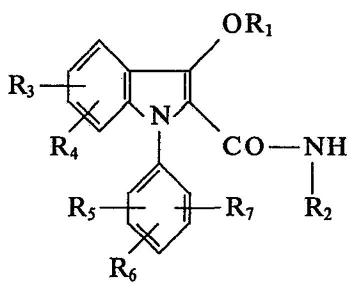

(IVa)

dans laquelle $R_1$, $R_2$, $R_3$, $R_4$, $R_5$, $R_6$ et $R_7$ ont la signification précédente

avec des composés de formule VIII

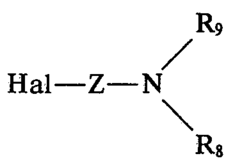

(VIII)

dans laquelle Z, $R_8$ et $R_9$ ont la signification précédente et Hal représente un halogène, ou que,

d) pour la préparation de composés de formule Ia

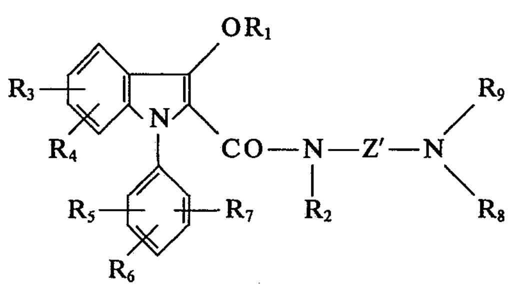

(Ia)

dans laquelle $R_1$, $R_2$, $R_3$, $R_4$, $R_5$, $R_6$, $R_7$, $R_8$ et $R_9$ ont la signification précédente et Z' représente un groupe alkylène avec 2 à 5 atomes de carbone qui est substitué par un hydroxy sur un atome de carbone non lié à de l'azote,

on fait réagir des composés de formule IV

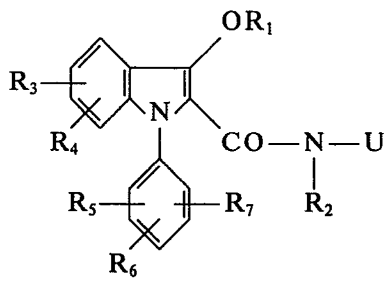

(IV)

dans laquelle $R_1$, $R_2$, $R_3$, $R_4$, $R_5$, $R_6$ et $R_7$ ont la signification précédente et U est l'hydrogène ou un groupe de formule V

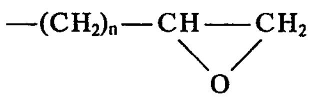

(V)

où n représente 1 à 3,

avec un composé de formule IX

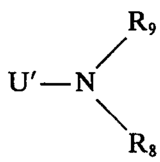

(IX)

dans laquelle $R_8$ et $R_9$ ont la signification précédente et U', dans le cas où U représente l'hydrogène, est un groupe de formule V ou, dans le cas où U représente un groupe de formule V, est l'hydrogène,

et que, le cas échéant, on transforme des composés libres de formule I en leurs sels d'addition avec des acides ou les sels d'addition avec des acides en les composés libres de formule I.

12. Médicament renfermant une quantité pharmacologiquement efficace d'un composé de 1-phenyl-2-aminocarbonylindole selon la revendication 1 et les adjuvants et/ou véhicules usuels.

13. Procédé pour la préparation de composés de formule générale IId

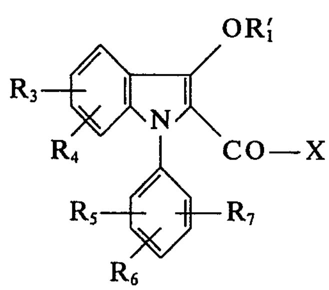

(IId)

dans laquelle $R'_1$, $R_3$, $R_4$, $R_5$, $R_6$, $RT_7$ et X ont la signification indiquée dans la revendication 9, caractérisé en ce que l'on cyclise

a') des composés de formule X

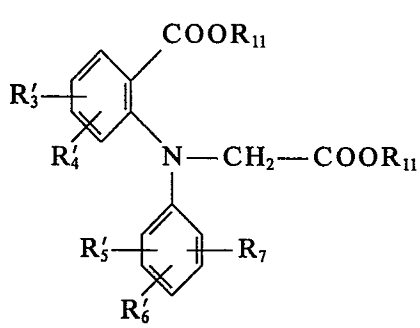

(X)

dans laquelle $R_7$ a la signification précédente et $R_{11}$ représente un alkyle avec 1 à 4 atomes de carbone, $R'_3$, $R'_4$, $R'_5$ et $R'_6$ possèdent dans chaque cas la signification indiquée à propos de $R_3$, $R_4$, $R_5$ et $R_6$, auquel cas un groupe hydroxy libre est toutefois doté d'un groupe protecteur, en des composés de formule IIb

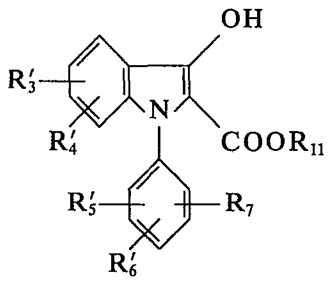

(IIb)

dans laquelle $R'_3$, $R'_4$, $R'_5$, $R'_6$, $R_7$ et $R_{11}$ ont la signification précédente et qu'on éthérifie ceux-ci en des composés de formule IIc

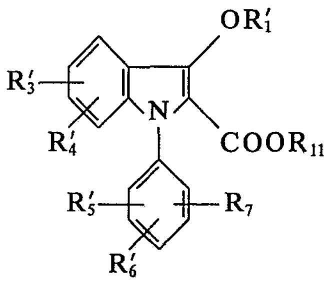

(IIc)

dans laquelle $R'_1$, $R'_3$, $R'_4$, $R'_5$, $R'_6$, $R_7$ et $R_{11}$ ont la signification précédente ou que,

b') pour la préparation de composés de formule IIc, on fait réagir des composés de formule XIa

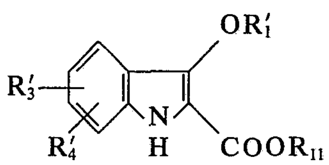

(XIa)

dans laquelle $R'_1$, $R'_3$, $R'_4$ et $R_{11}$ ont la signification précédente,

avec des composés de formule XII

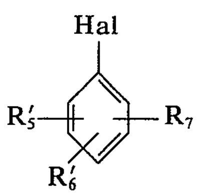

(XII)

dans laquelle $R'_5$, $R'_6$, $R_7$ et Hal ont la signification précédente, élimine les groupes protecteurs d'hydroxy éventuels, hydrolyse au besoin les esters de formule IId obtenus où X représente le groupe $OR_{11}$ en les acides correspondants de formule IId et transforme ceux-ci, le cas échéant, en leurs dérivés d'acides.

**Revendications pour l'Etat contractant: AT**

1. Procédé pour la préparation de composés de 1-phényl-2-aminocarbonylindole de formule générale I

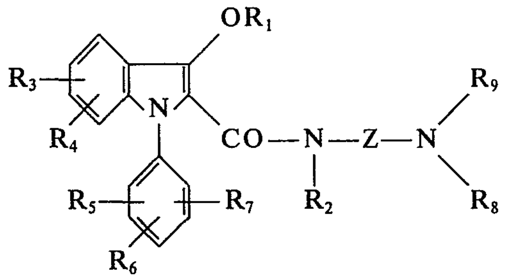

(I)

dans laquelle

| | |
|---|---|
| $R_1$ | représente l'hydrogène, un groupe alkyle, alcényle, cycloalkyle ou cycloalkylalkyle avec jusqu'à 7 atomes de carbone, |
| $R_2$ | représente l'hydrogène ou un alkyle avec 1 à 4 atomes de carbone, |
| $R_3$ | représente l'hydrogène, un halogène, un alkyle avec 1 à 4 atomes de carbone, un hydroxy ou un alcoxy avec 1 à 4 atomes de carbone, |
| $R_4$ | représente l'hydrogène, un halogène, un alkyle avec 1 à 4 atomes de carbone, un hydroxy, un alcoxy avec 1 à 4 atomes de carbone ou, au cas où $R_3$ est l'hydrogène, également un nitro ou un trifluorométhyle ou |
| $R_3$ et $R_4$ | sont fixés sur des atomes de carbone voisins et représentent ensemble un méthylènedioxy ou un éthylènedioxy, |
| $R_5$ | représente l'hydrogène, un alkyle avec 1 à 4 atomes de carbone, un halogène, un hydroxy ou un alcoxy avec 1 à 4 atomes de carbone, |
| $R_6$ | représente l'hydrogène, un alkyle avec 1 à 4 atomes de carbone, un alcoxy avec 1 à 4 atomes de carbone, un halogène, un hydroxy ou, au cas où $R_5$ est l'hydrogène, également un nitro ou un trifluorométhyle ou |
| $R_5$ et $R_6$ | sont fixés sur des atomes de carbone voisins et représentent ensemble un méthylènedioxy ou un éthylènedioxy, |
| $R_7$ | représente l'hydrogène ou, au cas où $R_5$ et $R_6$ sont des alcoxy avec 1 à 4 atomes de carbone, également un alcoxy avec 1 à 4 atomes de carbone, |
| $R_8$ | représente l'hydrogène ou un alkyle avec 1 à 4 atomes de carbone, |
| $R_9$ | représente l'hydrogène ou un alkyle avec 1 à 4 atomes de carbone ou |
| $R_8$ et $R_9$ | représentent ensemble, avec l'atome d'azote auquel ils sont liés, un groupe hétérocyclique a |

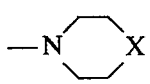

(a)

| | |
|---|---|
| | où X est une liaison, $-CH_2-$, $-C_2H_4-$, O ou S, |
| Z | représente une chaîne alkylène avec 2 à 5 atomes de carbone qui est éventuellement substituée par un hydroxy sur un carbone non lié à de l'azote, |

ainsi que leurs sels d'addition avec des acides, caractérisé en ce que l'on fait réagir

a) des composés de formule II

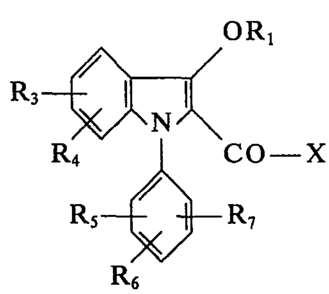

(II)

dans laquelle $R_1$, $R_3$, $R_4$, $R_5$, $R_6$ et $R_7$ ont la signification précédente et X représente un hydroxy ou un groupe réactif,

avec un composé de formule VI

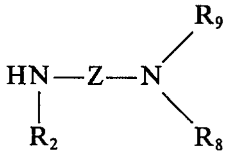

(VI)

dans laquelle $R_2$, $R_8$, $R_9$ et Z ont la signification précédente, ou que l'on fait réagir

b) des composés de formule III

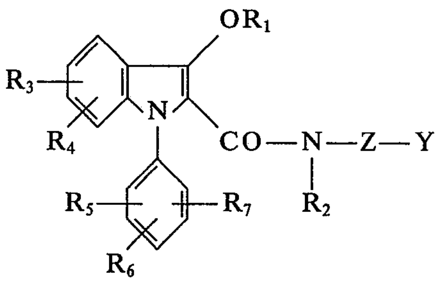

(III)

dans laquelle $R_1$, $R_2$, $R_3$, $R_4$, $R_5$, $R_6$, $R_7$ et Z ont la signification précédente et Y représente un groupe éliminable par aminolyse,

avec des composés de formule VII

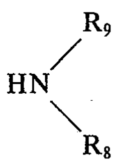

(VII)

dans laquelle $R_8$ et $R_9$ ont la signification précédente, ou que l'on fait réagir

c) des composés de formule IVa

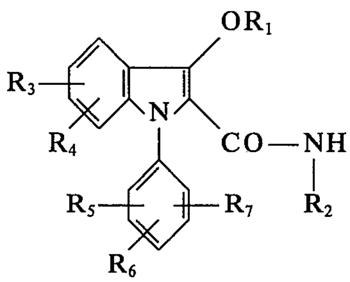

(IVa)

dans laquelle $R_1$, $R_2$, $R_3$, $R_4$, $R_5$, $R_6$ et $R_7$ ont la signification précédente,

avec des composés de formule VIII

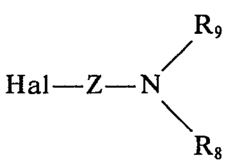

(VIII)

dans laquelle Z, $R_8$ et $R_9$ ont la signification précédente et Hal représente un halogène, ou que,

d) pour la préparation de comosés de formule Ia

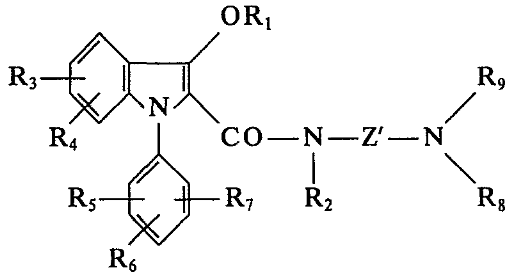

(Ia)

dans laquelle $R_1$, $R_2$, $R_3$, $R_4$, $R_5$, $R_6$, $R_7$, $R_8$ et $R_9$ ont la signification précédente et Z' représente un groupe alkylène avec 2 à 5 atomes de carbone qui est substitué par un hydroxy sur un atome de carbone non lié à de l'azote,

on fait réagir des composés de formule IV

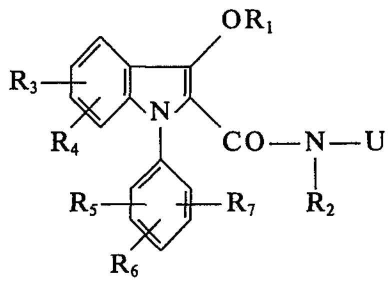

(IV)

dans laquelle $R_1$, $R_2$, $R_3$, $R_4$, $R_5$, $R_6$ et $R_7$ ont la signification précédente et U est l'hydrogène ou un groupe de formule V

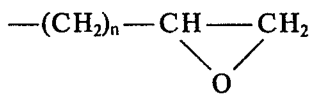

(V)

où n représente 1 à 3,

avec un composé de formule IX

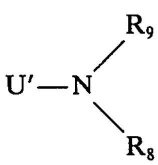

(IX)

dans laquelle $R_8$ et $R_9$ ont la signification précédente et U', dans le cas où U représente l'hydrogène, est un groupe de formule V, ou, dans le cas où U représente un groupe de formule V, est l'hydrogène,

et que, le cas échéant, on transforme des composés libres de formule I en leurs sels d'addition avec des acides ou les sels d'addition avec des acides en les composés libres de formule I.

2. Procédé selon la revendication 1, caractérisé en ce que l'on prépare en tant que composés de formule I des composés de 1-phényl-2-aminocarbonylindole, dans lesquels Z et $R_3$ à $R_9$ ont la signification précédente, $R_1$ représente un alkyle avec 1 à 4 atomes de carbone ou un cycloalkylalkyle et $R_2$ l'hydrogène.

3. Procédé selon la revendication 2, caractérisé en ce que l'on prépare, en tant que composés de formule I, des composés de 1-phényl-2-aminocarbonylindole, dans lesquels Z et $R_1$ à $R_7$ ont la signification précédente, $R_8$ représente l'hydrogène ou un alkyle avec 1 à 4 atomes de carbone et $R_9$ représente l'hydrogène ou un alkyle avec 1 à 4 atomes de carbone.

4. Procédé selon la revendication 1, caractérisé en ce que l'on prépare en tant que composés de formule I, des composés de 1-phenyl-2-aminocarbonylindole, dans lesquels Z a la signification précédente, $R_1$ représente l'hydrogène ou un alkyle avec 1 à 4 atomes de carbone, $R_2$ représente l'hydrogène, $R_3$ représente l'hydrogène ou un halogène, $R_4$ représente l'hydrogène ou un halogène, $R_5$ représente l'hydrogène, un halogène ou un alkyle avec 1 à 4 atomes de carbone, $R_6$ représente l'hydrogène, un

halogène ou un alkyle avec 1 à 4 atomes de carbone, $R_7$ représente l'hydrogène, $R_8$ représente l'hydrogène ou un alkyle avec 1 à 4 atomes de carbone et $R_9$ représente l'hydrogène ou un alkyle avec 1 à 4 atomes de carbone.

5. Procédé selon la revendication 4, caractérisé en ce que l'on prépare, en tant que composés de formule I des composés de 1-phényl-2-aminocarbonylindole, dans lesquels $R_1$ à $R_9$ ont la signification précédente et Z représente une chaîne alkylène avec 2 à 5 atomes de carbone qui est substituée par un hydroxy sur un atome de carbone non lié à de l'azote.

6. Procédé selon la revendication 5, caractérisé en ce que l'on prépare, en tant que composés de formule I le 5-bromo-2-[3-(N,N-diéthylamino)-2-hydroxypropylaminocarbonyl]-3-méthoxy-1-phénylindole et ses sels d'addition avec des acides.

7. Procédé selon la revendication 5, caractérisé en ce que l'on prépare en tant que composés de formule I le 2-[3-(N,N-diéthylamino)-2-hydroxypropylaminocarbonyl]-3-hydroxy-1-phénylindole et ses sels d'addition avec des acides.

8. Procédé selon la revendication 5, caractérisé en ce que l'on prépare, en tant que composés de formule I le 2-[3-(N,N-diéthylamino)-2-hydroxypropylaminocarbonyl]-3-méthoxy-1-phénylindole et ses sels d'addition avec des acides.

9. Procédé pour la préparation de composés de formule générale IId

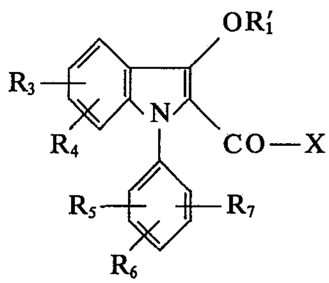

(IId)

dans laquelle $R_1'$ représente un groupe alkyle, alcényle, cycloalkyle ou cycloalkylalkyle avec jusqu'à 7 atomes de carbone et $R_1$, $R_4$, $R_5$, $R_6$, $R_7$ et X ont la signification indiquée dans la revendication 1, caractérisé en ce que l'on cyclise

a') des composés de formule X

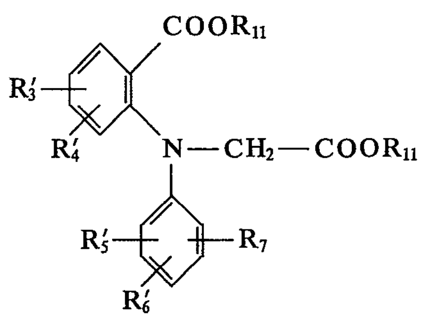

(X)

dans laquelle $R_7$ a la signification précédente et $R_{11}$ représente un alkyle avec 1 à 4 atomes de carbone, $R_3'$, $R_4'$, $R_5'$ et $R_6'$ possèdent dans chaque cas la signification indiquée à propos de $R_3$, $R_4$, $R_5$ et $R_6$, auquel cas un groupe hydroxy libre est toutefois doté d'un groupe protecteur, en des composés de formule IIb

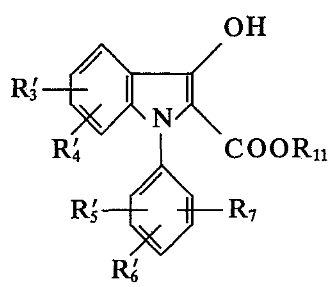

(IIb)

dans laquelle $R'_3$, $R'_4$, $R'_5$, $R'_6$, $R_7$ et $R_{11}$ ont la signification précédente et qu'on éthérifie ceux-ci en des composés de formule IIc

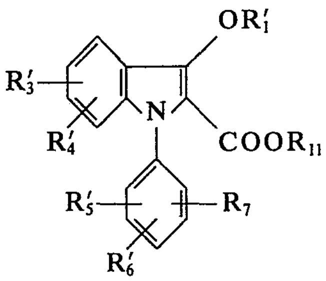

(IIc)

dans laquelle $R'_1$, $R'_3$, $R'_4$, $R'_5$, $R'_6$, $R_7$ et $R_{11}$ ont la signification précédente ou que,

b') pour la préparation de composés de formule IIc, on fait réagir des composés de formule XIa

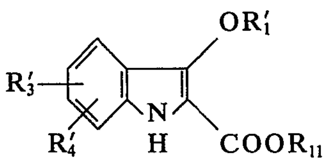

(XIa)

dans laquelle $R'_1$, $R'_3$, $R'_4$ et $R_{11}$ ont la signification précédente,

avec des composés de formule XII

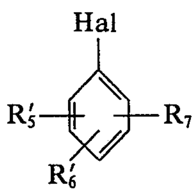

(XII)

dans laquelle $R'_5$, $R'_6$, $R_7$ et Hal ont la signification précédente, élimine les groupes protecteurs d'hydroxy éventuels, hydrolyse au besoin les esters de formule IId obtenus où X représente le groupe $OR_{11}$, en les acides correspondants de formule IId et transforme ceux-ci, le cas échéant, en leurs dérivés d'acides.

10. Procédé selon la revendication 9, caractérisé en ce que l'on prépare des composés de formule IId, dans laquelle $R'_1$, $R_3$, $R_4$, $R_5$, $R_6$ et $R_7$ ont la signification précédente et X est un hydroxy, un halogène, un alcoxy avec 1 à 4 atomes de carbone ou un groupe O—CO—W où W représente un alkyle inférieur ou un alcoxy inférieur.